# EUROPEAN PATENT APPLICATION

(11) **EP 3 973 990 A1**
(43) Date of publication of application: **30.03.2022**
(21) Application number: 20779344.9
(22) Date of filing: 19.03.2020
(51) Int. Cl.: A61K 45/00, A61K 47/42

(54) **PROMOTOR FOR PERMEATION INTO CELL LAYER, COMPOSITION FOR FACILITATING DRUG AGENT ABSORPTION, AND PHARMACEUTICAL COMPOSITION**

(30) Priority: 25.03.2019 JP 2019057323
(71) Applicant: University of Tsukuba, Ibaraki 305-8577 (JP)
(72) Inventor: USUI Takeo, Tsukuba-shi, Ibaraki 305-8577 (JP); NAGUMO Yoko, Tsukuba-shi, Ibaraki 305-8577 (JP); MUKAIYAMA Minagi, Tsukuba-shi, Ibaraki 305-8577 (JP); HAYASHI Yoshio, Hachioji-shi, Tokyo 192-0392 (JP); TANIGUCHI Atsuhiko, Hachioji-shi, Tokyo 192-0392 (JP); UCHIYAMA Chihiro, Hachioji-shi, Tokyo 192-0392 (JP)
(74) Representative: Mewburn Ellis LLP
(86) International application number: PCT/JP2020/012446
(87) International publication number: WO 2020/196315

(57) **Abstract**

There is provided technology for promoting permeation of a substance into a cell layer. Provided is a cell layer permeation promoter comprising a compound represented by general formula (1). Also provided is a composition for facilitating drug absorption that contains the cell layer permeation promoter, said composition being for facilitating absorption of a drug into a living body. Also provided is a pharmaceutical composition that contains the cell layer permeation promoter and furthermore contains a drug to be absorbed into a living body.

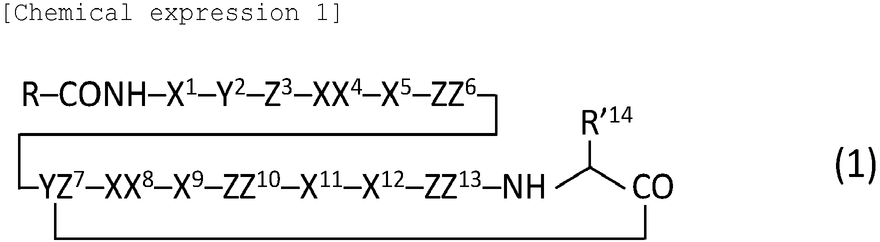

## Description

### TECHNICAL FIELD

The present invention relates to a cell layer permeation promoter, a composition for facilitating drug absorption, and a pharmaceutical composition.

### BACKGROUND ART

In cell layers forming epithelial tissues, etc., gaps between adjacent cells are sealed by tight junctions (TJ) or other cell bonding structures, fulfilling a function as a barrier against foreign matter. Therefore, it can be said that permeation of substances in paracellular pathways, which pass between cells, is extremely limited in order to maintain homeostasis of living bodies. There is a mode of thinking pertaining to drug delivery systems (DDSs), in which the cell bonding structures are preliminarily relaxed and a desired substance is caused to pass through the paracellular pathways in an attempt to improve absorption of drugs, etc., into living bodies (Non-Patent Documents 1 to 5). Among biopharmaceutical products that have been widely developed in recent years and candidate substances therefor, such as peptides, proteins, antibodies, vaccines, and nucleic acids, some have low bioavailability. The technology of drug delivery systems (DDSs) increases the range of applicability of such drugs and leads to improvements in patients' quality of life due to non-invasive administration methods such as oral, percutaneous, pernasal, pulmonary, and transmucosal administration rather than administration through injection. Therefore, there is a demand to further develop this technology.

Cyclic lipopeptides are reported as Pseudomonas-derived antimicrobial substances in Non-Patent Document 6.

### [Related Art Documents]

### [Non-Patent Documents]

[Non-Patent Document 1] Kondoh M., et al. "Tight junction modulators: Promising candidates for drug delivery." Current Medicinal Chemistry. (2007) 14(23), pp. 2482-2488.
[Non-Patent Document 2] Gonzalez-Mariscal L., et al. "Strategies that target tight junctions for enhanced drug delivery." Current Pharmaceutical Design. (2016) 22(35), pp. 5313-5346.
[Non-Patent Document 3] Eichner M., et al. "Targeting and alteration of tight junctions by bacteria and their virulence factors such as Clostridium perfringens enterotoxin." Pflugers Archiv: European Journal of Physiology. (2017) 469(1), pp. 77-90.
[Non-Patent Document 4] Fujii Yoshikazu. "Ampicillin suppository." Japanese Journal of Antibiotics. (1986) 39, pp. 1-8.
[Non-Patent Document 5] Yamamoto Akira. "Improvement of transmucosal delivery of peptide and protein drugs." Journal of Pharmaceutical Science and Technology, Japan. (2014) 74(1), pp. 19-26.
[Non-Patent Document 6] Wen Li, et al. "The antimicrobial compound xantholysin defines a new group of Pseudomonas cyclic lipopeptides." PLoS One. (2013) 8(5), e62946.

### DISCLOSURE OF THE INVENTION

### [Problems the Invention is Intended to Solve]

However, Non-Patent Document 6 is not focused on technology for promoting permeation of a substance into a cell layer.

It is accordingly an object of the present invention to provide technology for promoting permeation of a substance into a cell layer.

### [Means for Solving the Problems]

As a result of thorough investigations directed toward achieving this object, the inventors discovered that a specific cyclic lipopeptide has an effect for promoting permeation of a substance into a cell layer.

Specifically, in a first aspect, the present invention provides a cell layer permeation promoter comprising a compound represented by general formula (1). 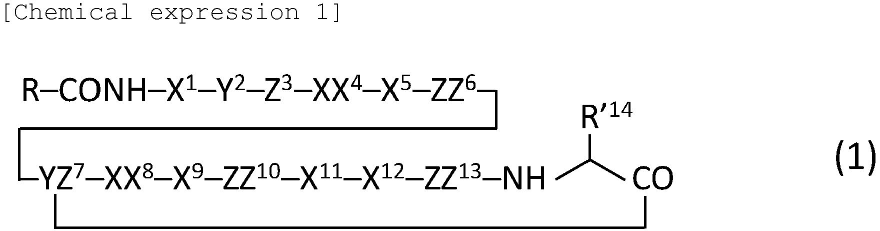
(In general formula (1): R is a C1-9 alkyl group optionally having a substituent;
X is leucine or a conservative substitute therefor, and is more preferably leucine, isoleucine, norleucine, tert-butyl alanine, tert-leucine, valine, cyclohexyl glycine or cyclohexyl alanine, or alanine;
XX is valine or a conservative substitute therefor, and is more preferably valine, isoleucine, norleucine, tert-butyl alanine, tert-leucine, leucine, cyclohexyl glycine or cyclohexyl alanine, or alanine;
Y is glutamic acid or a conservative substitute therefor, and is more preferably glutamic acid or aspartic acid, or alanine;
Z is glutamine or a conservative substitute therefor, and is more preferably glutamine or asparagine, or alanine;
ZZ is a discretionary α-amino acid, and is more preferably glutamine or alanine;
YZ is serine, threonine, homoserine, diaminopropanoic acid, or diaminobutyric acid; and
R' is sec-butyl or a conservative substitute therefor, and is more preferably sec-butyl, isopropyl, isopentyl, n-butyl, tert-butyl, cyclohexyl, or cyclohexyl methyl.)

In the present invention, the compound represented by general formula (1) is preferably a compound represented by general formula (2). 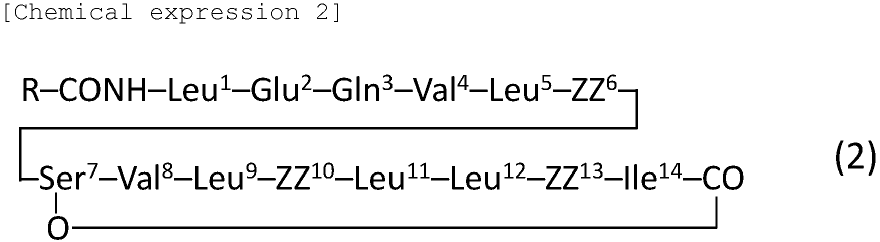
(In general formula (2): R is a C1-9 alkyl group optionally having a substituent; and
ZZ is a discretionary α-amino acid, and is more preferably glutamine or alanine.)

In a second aspect, the present invention provides a composition for facilitating drug absorption that contains the cell layer permeation promoter, said composition being for facilitating absorption of a drug into a living body.

In a third aspect, the present invention provides a pharmaceutical composition that contains the cell layer permeation promoter and furthermore contains a drug to be absorbed into a living body.

### [Effect of the Invention]

According to the present invention, because a specific cyclic polypeptide has an effect for promoting permeation of a substance into a cell layer, it is possible to use this cyclic polypeptide to provide a cell layer permeation promoter, a composition for facilitating absorption of a drug, and a pharmaceutical composition, those are exceptional.

### BRIEF DESCRIPTION OF THE DRAWINGS

FIG. 1 is a graph showing the result, in test example 1, of evaluating the effects of various cyclic polypeptides on the permeation of dextran having a molecular weight of 4000 in a permeation evaluation test in which a model cell layer is used;
FIG. 2 is a graph showing the result, in test example 2, of evaluating the effects of various cyclic polypeptides on the permeation of dextran having a molecular weight of 4000 in a permeation evaluation test in which a model cell layer is used;
FIG. 3 is a graph showing the result, in test example 3, of evaluating the effects of various cyclic polypeptides on the permeation of dextran having a molecular weight of 4000 in a permeation evaluation test in which a model cell layer is used; and
FIG. 4 is a graph showing the result, in test example 4, of evaluating the effects of various cyclic polypeptides on the permeation of dextran having a molecular weight of 4000 in a permeation evaluation test in which a model cell layer is used.

### MODE FOR CARRYING OUT THE INVENTION

In the present invention, a compound represented by general formula (1) is used. 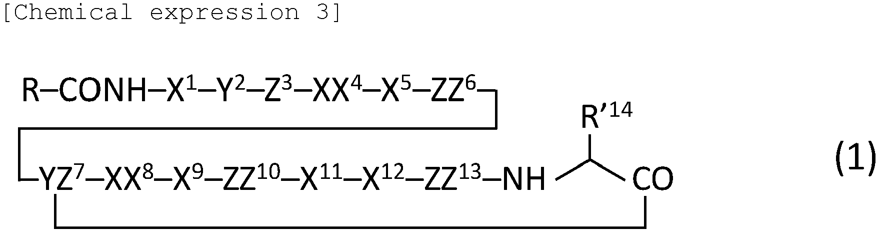
(In general formula (1): R is a C1-9 alkyl group optionally having a substituent;
X is leucine (Leu) or a conservative substitute therefor, and is more preferably leucine (Leu), isoleucine (Ile), norleucine (Nle), tert-butyl alanine (Ala{tBu}), tert-leucine (tert-Leu), valine (Val), cyclohexyl glycine (Chg) or cyclohexyl alanine (Cha), or alanine (Ala);
XX is valine (Val) or a conservative substitute therefor, and is more preferably valine (Val), isoleucine (Ile), norleucine (Nle), tert-butyl alanine (Ala{tBu}), tert-leucine (tert-Leu), leucine (Leu), cyclohexyl glycine (Chg) or cyclohexyl alanine (Cha) , or alanine (Ala);
Y is glutamic acid (Glu) or a conservative substitute therefor, and is more preferably glutamic acid (Glu) or aspartic acid (Asp) , or alanine (Ala);
Z is glutamine (Gln) or a conservative substitute therefor, and is more preferably glutamine (Gln) or asparagine (Asn) , or alanine (Ala);
ZZ is a discretionary α-amino acid, and is more preferably glutamine (Gln) or alanine (Ala);
YZ is serine (Ser), threonine (Thr), homoserine, diaminopropanoic acid, or diaminobutyric acid; and
R' is sec-butyl or a conservative substitute therefor, and is more preferably sec-butyl, isopropyl, isopentyl, n-butyl, tert-butyl, cyclohexyl, or cyclohexyl methyl.)

The compound in general formula (1) when R' is sec-butyl has a configuration in which the α-amino groups and α-carboxyl groups in isoleucine (Ile) form peptide bonds with the adjacent amino acid. Similarly, the compound has a configuration in which the peptide bonds with the adjacent amino acid are formed, respectively, by valine (Val) when R' is isopropyl, by leucine (Leu) when R' is isopentyl, by norleucine (Nle) when R' is n-butyl, by tert-butyl alanine (Ala{tBu}) when R' is tert-butyl, by cyclohexyl glycine (Chg) when R' is cyclohexyl, and by cyclohexyl alanine (Cha) when R' is cyclohexyl methyl.

More specifically, in the present invention, the compound represented by general formula (1) may be a compound represented by general formula (2). 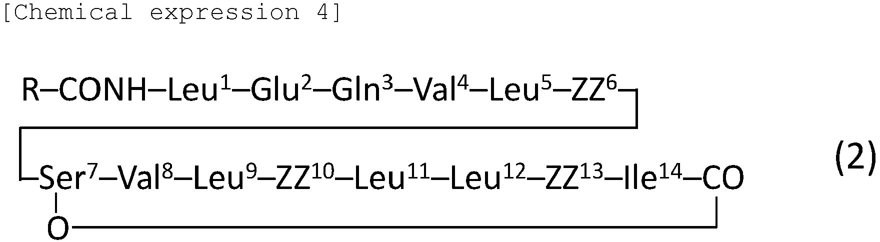
(In general formula (2): R is a C1-9 alkyl group optionally having a substituent; and
ZZ is a discretionary α-amino acid, and is more preferably glutamine or alanine.)

With respect to the compound represented by general formula (2):
(A) a case in which ZZ⁶ is alanine (Ala), ZZ¹⁰ is glutamine (Gln), and ZZ¹³ is glutamine (Gln) is represented in more detail by chemical structure (2a);
(B) a case in which ZZ⁶ is glutamine (Gln), ZZ¹⁰ is alanine (Ala), and ZZ¹³ is glutamine (Gln) is represented in more detail by chemical structure (2b);
(C) a case in which ZZ⁶ is glutamine (Gln), ZZ¹⁰ is glutamine (Gln), and ZZ¹³ is alanine (Ala) is represented in more detail by chemical structure (2c); and
(D) a case in which ZZ⁶ is glutamine (Gln), ZZ¹⁰ is glutamine (Gln), and ZZ¹³ is glutamine (Gln) is represented in more detail by chemical structure (2d).

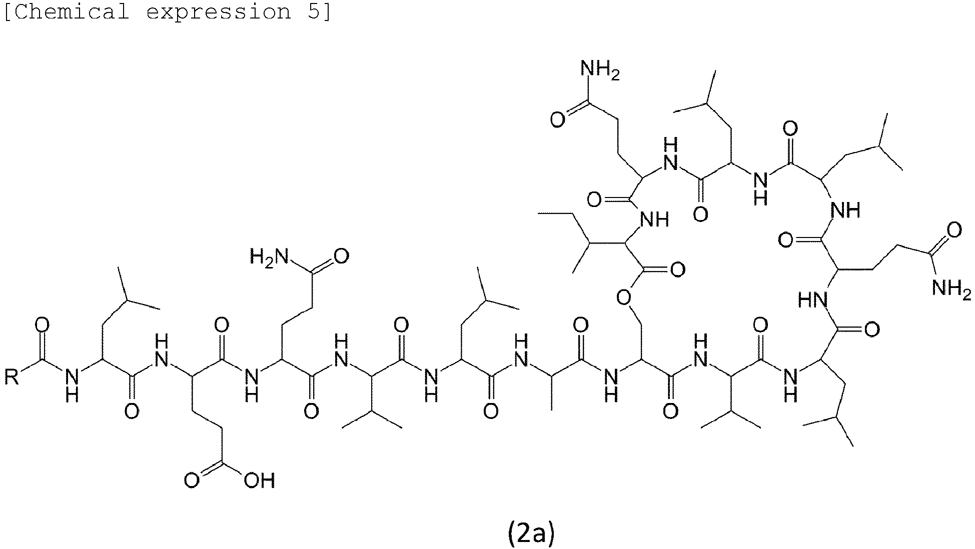

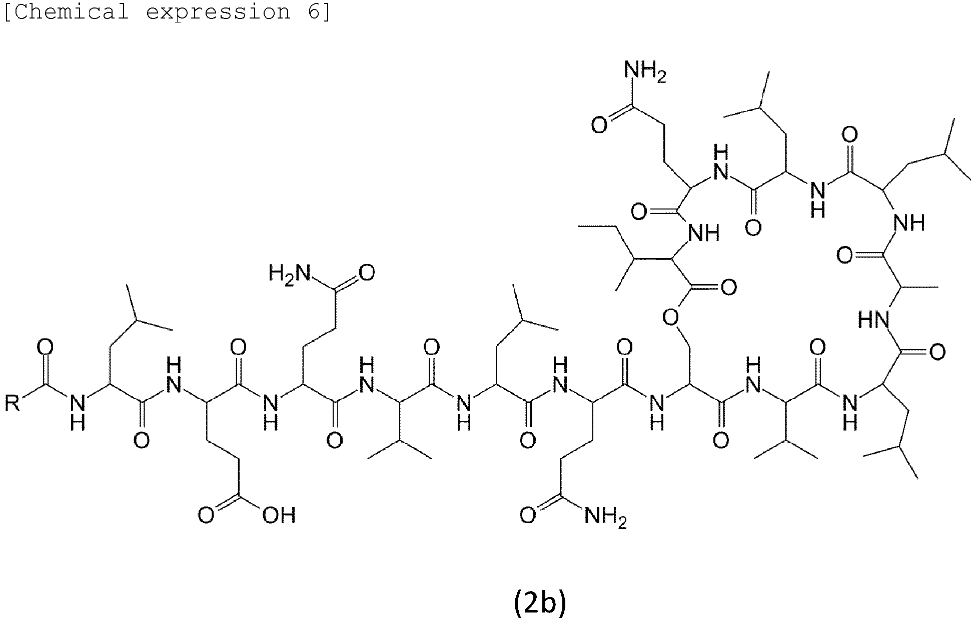

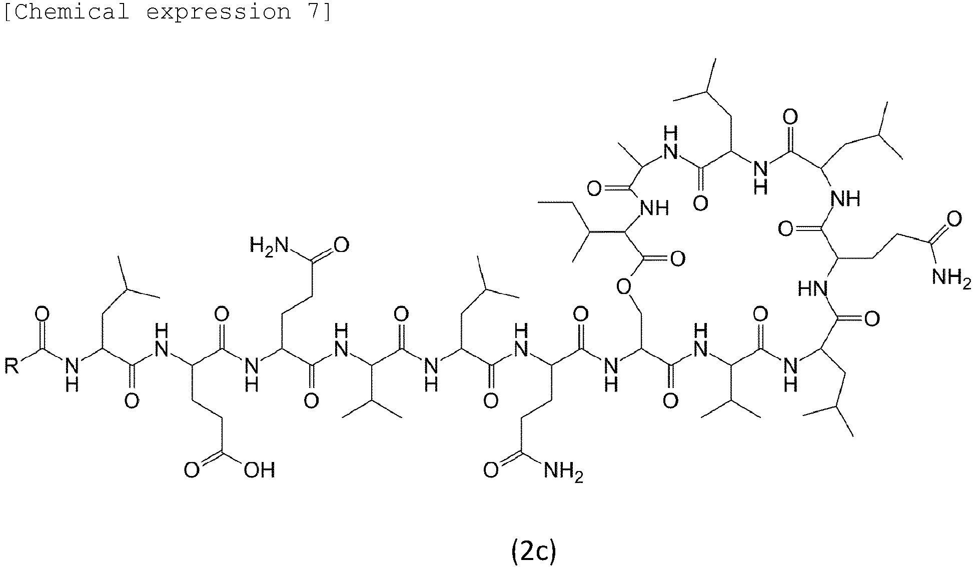

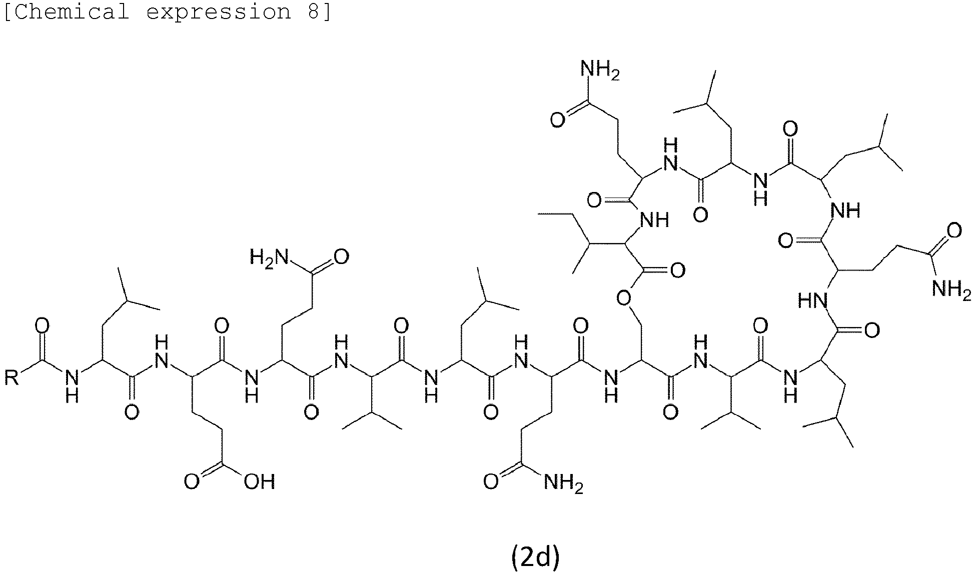

The compound represented by general formula (1) may be a compound represented by general formula (3), in which the DL isomer structure of the peptide bonds is specified. 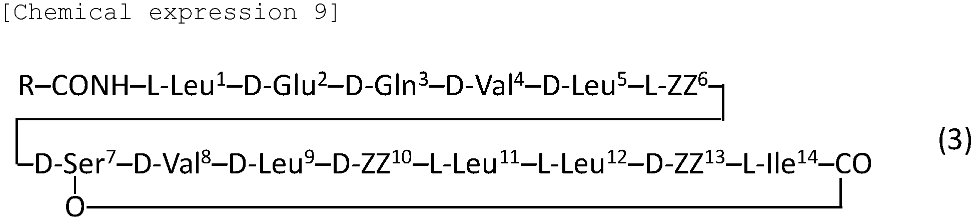
(In general formula (3): R is a C1-9 alkyl group optionally having a substituent; and
ZZ is a discretionary α-amino acid, and is more preferably glutamine or alanine.)

With respect to the compound represented by general formula (3):
(A) a case in which ZZ⁶ is alanine (Ala), ZZ¹⁰ is glutamine (Gln), and ZZ¹³ is glutamine (Gln) is represented in more detail by chemical structure (3a);
(B) a case in which ZZ⁶ is glutamine (Gln), ZZ¹⁰ is alanine (Ala), and ZZ¹³ is glutamine (Gln) is represented in more detail by chemical structure (3b);
(C) a case in which ZZ⁶ is glutamine (Gln), ZZ¹⁰ is glutamine (Gln), and ZZ¹³ is alanine (Ala) is represented in more detail by chemical structure (3c); and
(D) a case in which ZZ⁶ is glutamine (Gln), ZZ¹⁰ is glutamine (Gln), and ZZ¹³ is glutamine (Gln) is represented in more detail by chemical structure (3d).

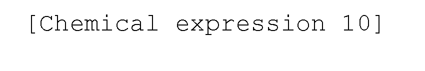

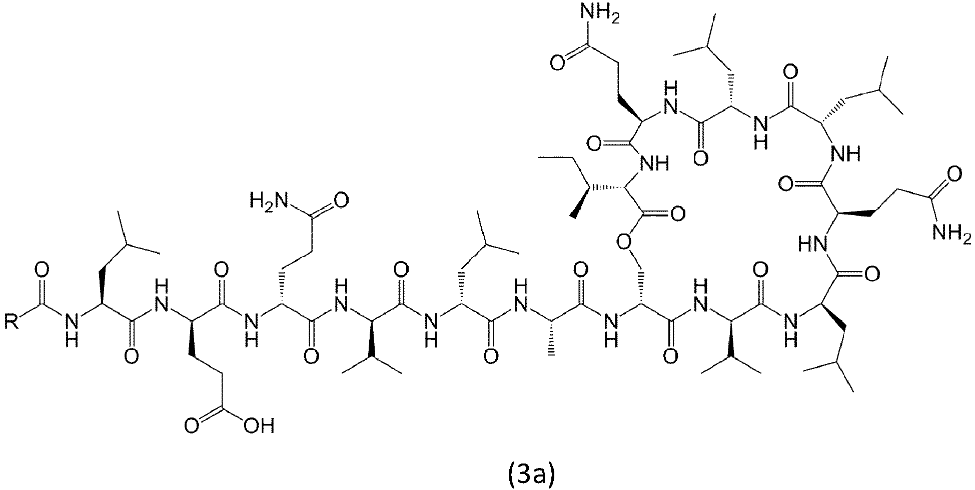

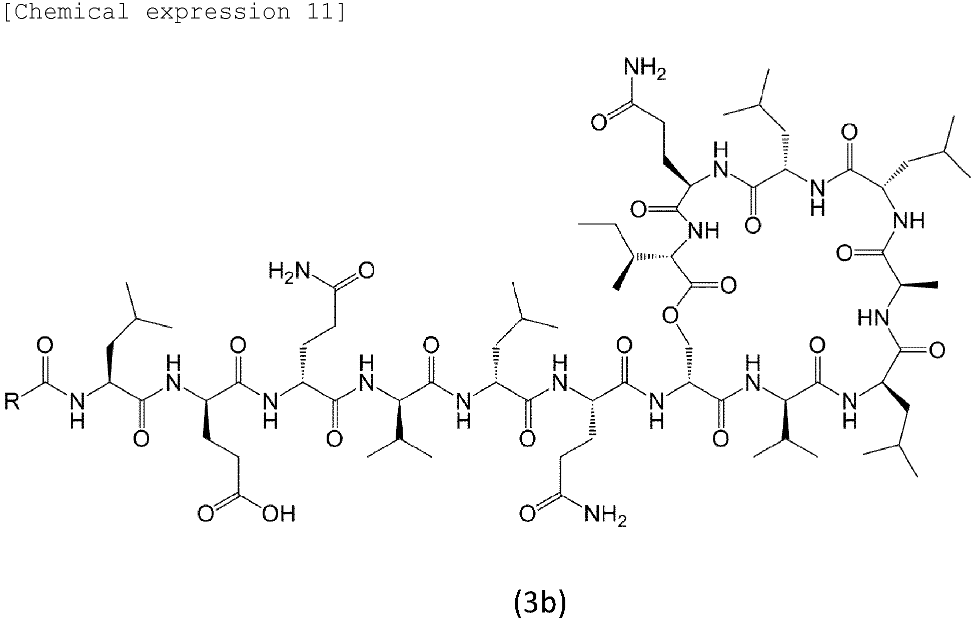

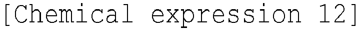

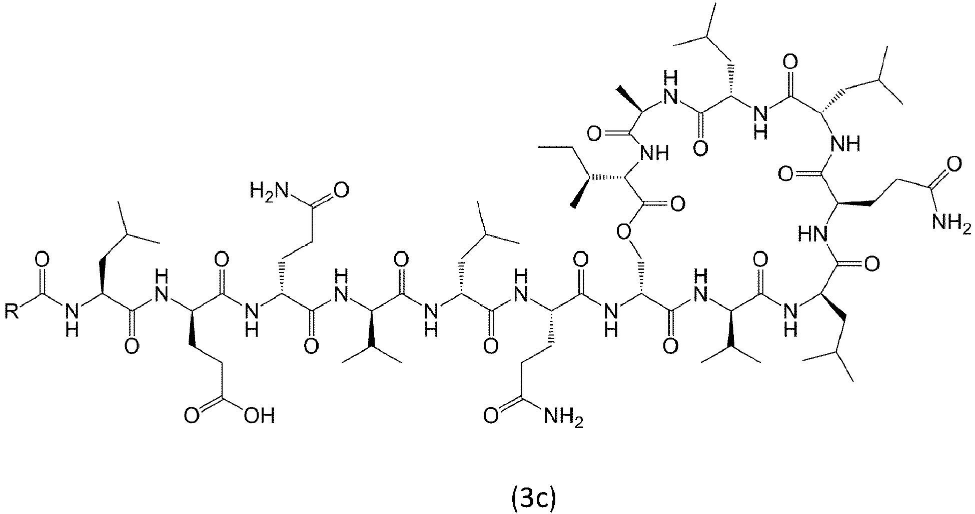

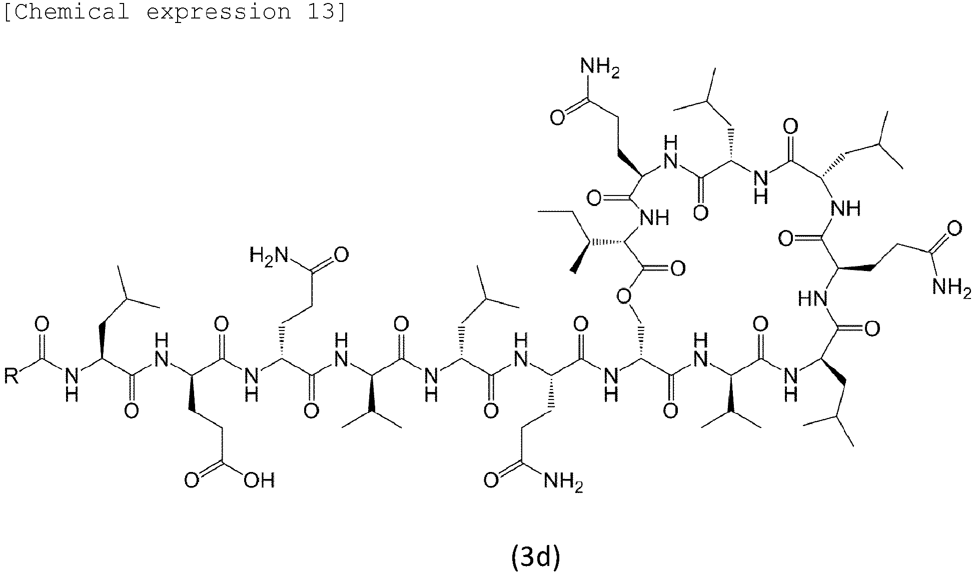

In the general formula for the abovementioned compound, R is represented as a C1-9 alkyl group optionally having a substituent. The alkyl group typically may have a hydroxyl group as desired, more typically may be an alkyl group having only one to four hydroxyl groups as substituents, and even more typically may be an alkyl group having only one to two hydroxyl groups as substituents. The alkyl group may have a length of C1-6, or may have a length of C1-3. Furthermore, in consideration of the examples (described below), it is more typical for R to be a group represented by formula (4) or (5). The alkyl groups represented by formulas (4) and (5) have a length of C9, but these alkyl groups may have a length of C6, or may have a length of C3.

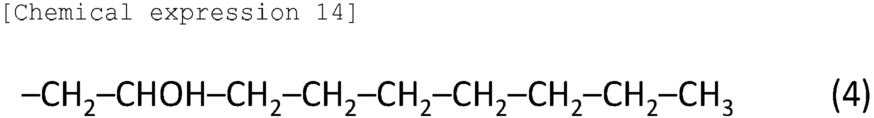

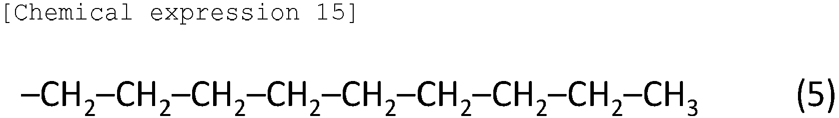

It is also clear that at least one type of the compound used in the present invention is included in a certain type of Pseudomonas, as indicated in Non-Patent Document 6. The compound used in the present invention may be prepared by extraction from such a bacterial strain, a mutant strain, a transgenic strain, etc., or may be prepared by extraction from another bioresource such as other microorganisms; however, from the standpoint of industrially producing the compound, it is more preferable for the compound to be artificially synthesized. Examples of the method of synthesis include liquid-phase methods, Ajiphase methods, and solid-phase methods. Among these, synthesis through solid-phase methods are more preferred because refining of intermediates can be dispensed with.

The specific example of Fmoc solid-phase synthesis is described below as one example of a solid-phase method. However, the disclosure of preferred synthesis methods indicated below is in no way intended to limit the scope of the present invention to compounds obtained through these methods.

### (Fmoc solid-phase synthesis)

As a procedure for Fmoc solid-phase synthesis, first Fmoc (9-fluorenylmethoxycarbonyl) groups, which are protective groups in resins, are reacted for 20 minutes at room temperature in a 20% (v/v) piperidine/DMF solution and removed. After cleansing of the resin, a variety of condensation agents may be used to condense the amino acids. This cycle is repeated to acquire a target peptide. As the condensation agents, carbodiimide reagents, diphenylphosphoryl azide, BOP reagents, HATU reagents, etc., may be used.

In the present invention, solid-phase synthesis can involve, e.g., synthesizing the thirteenth D-Gln residue from the N terminal as a starting point. Specifically, the Fmoc groups in the resin are deprotected by using a 20% (v/v) piperidine/DMF solution, and then side-chain unprotected Fmoc-D-Glu-Oallyl is condensed. Deprotection and condensation are repeated to carry out elongation to the eighth D-Val residue. After elongation, deprotection is carried out, and N(((9H-fluorene-9-yl)methoxy)carbonyl)-O-((2R)-2-(((allyloxy)carbonyl)amino)-3-methylpentanoyl)-D-serine is introduced into the resin. In order to form rings in the resin, a palladium catalyst is used to remove allyl groups and Alloc (allyloxy carbonyl) groups. Rings are then formed in the resin. Thereafter, the target peptide can be obtained by elongation from the C-terminal side in sequence in the same manner as with Fmoc-D-Glu-Oallyl.

In this method, various cyclic peptides can be synthesized in the resin by modifying the amino acid being condensed. For example, when the thirteenth D-Gln residue from the N terminal is to be modified to another amino acid, substitution can be carried out by modifying the position for bonding with the resin from the thirteenth to the tenth D-Gln.

The cell layer permeation promoter according to the present invention comprises the aforementioned compound, or the compound is used therefor. Specifically, as indicated in the examples (described below), when the compound acts on a cell layer, some type of effect is imparted to the cell bonding structure and permeation of a substance into a paracellular pathway is accelerated. In this case, specific examples of the application subject include the skin, the lungs, the nose, the alimentary canal, the digestive tract, and other mucous membrane tissues; there is no particular limitation as to the type of tissue. In the event of a form in which cells form a single layer together, and in the event of a form in which the cells form a multiple layer together, there is no particular limitation as to the type of layer shape thereof. In addition, the cell layer permeation promoter is not only for use on humans; cell layers derived from pets, livestock, and other animals may also be employed as subjects. Furthermore, in some circumstances, the cell layer permeation promoter may be applied to cell layers formed outside of living bodies, as necessary.

The composition for facilitating drug absorption according to the present invention is for facilitating absorption of a drug into a living body and contains a cell layer permeation promoter formed from the aforementioned compound. Specifically, by treating, e.g., the skin, the lungs, the nose, the alimentary canal, the digestive tract, or other mucous membrane tissues, as cell layers to be used as application subjects, using this composition together with a desired drug to be absorbed into a living body, the compound becomes an active ingredient and the substance, etc., in the paracellular pathway is accelerated, therefore making it possible to improve the absorption rate of the desired drug into the living body through said pathway. In this case, there are no particular limitations as to the form of the composition, provided that the compound is included therein. For example, it is possible to employ forms such as: salves, creams, lotions, milky lotions, and other endermic liniments; and patches, adhesive patches, aerosols, inhalants, suppositories, troches, sublingual tablets, orally disintegrating agents, jellies, liquids, soft capsules, hard capsules, powders, granules, and tablets, through formulation methods that are well known to persons skilled in the art together with a suitable formulative substrate in accordance with the type of cell layer to be used as an application subject. The compound content of the composition is, e.g., 0.0001-100 mass%, more typically 0.001-75 mass%, even more typically 0.01-50 mass%, particularly typically 0.1-30 mass%, and more particularly typically 1-20 mass%, etc.

Examples of drugs to which the present invention is applied include low-molecular compounds, peptides, proteins, antibodies, vaccines, and nucleic acids that are hydrophilic, lipophilic, or amphiphilic, there being no particular limitation as to such drugs. For example, the present invention is applied to substances having a molecular weight of 500 or greater, more preferably 1000 or greater, and even more preferably 2000 or greater, to improve the rate of absorption thereof into the living body. This increases the range of applicability of these substances as drugs and leads to improvements in patients' quality of life due to development of non-invasive administration methods such as oral, percutaneous, pernasal, pulmonary, and transmucosal administration rather than administration through injection, and therefore is preferred.

The form of a pharmaceutical composition in which the aforementioned compound and a desired drug to be absorbed into a living body are incorporated into the same form within the form of the composition for facilitating drug absorption may also be employed as another form achieved in the present invention.

The dosage of the compound according to the present invention differs depending on the type of cell layer to be used as an application subject and the type of drug to be absorbed into the living body. For example, although not as an unconditional rule, the application amount for one instance per square centimeter of the surface area of the cell layer may typically be about 0.01 µg to 10 mg, more typically about 0.1 µg to 1 mg, and even more typically about 1 µg to 0.1 mg. As indicated in the examples (described below), an action for opening cell gaps performed by the aforementioned compound is not an irreversible action, such as with an actin polymerization inhibitor that is known in the prior art, but rather is a reversible action; because the normal state of tight junctions and other cell bonding structures might recover relatively quickly after treatment due to an action for maintaining homeostasis of the living body, the present invention can be used safely without impairing the functioning as a barrier against foreign matter.

### [Examples]

The present invention is specifically described through the examples given below, but these examples do not in any way limit the scope of the present invention.

### [Preparation example 1] (Solid-phase synthesis of cu538-1 and cu538-2)

Through Fmoc solid-phase synthesis, the cu538-1 and cu538-2 illustrated below were synthesized. These compounds were obtained as racemic bodies in which isomers were present at the OH-group positions indicated by arrows in conjunction with the chemical structures below, and were prepared by separating and refining the racemic bodies through HPLC.

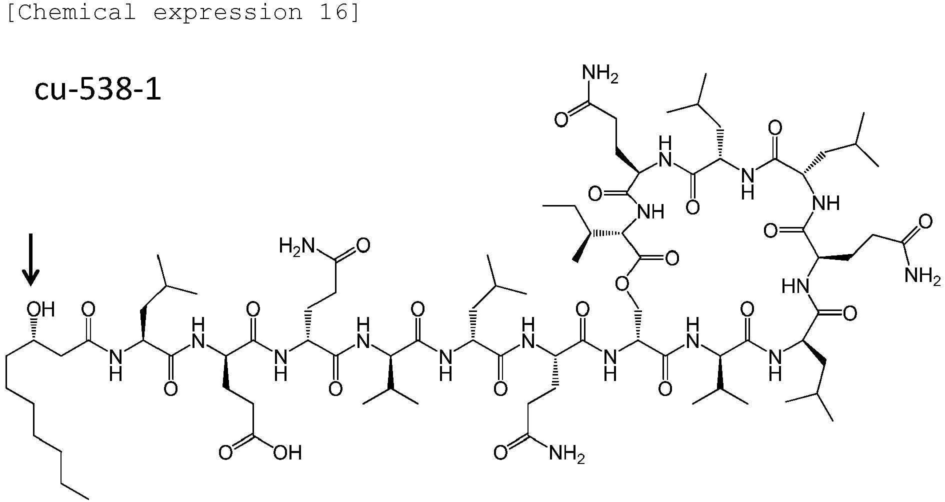

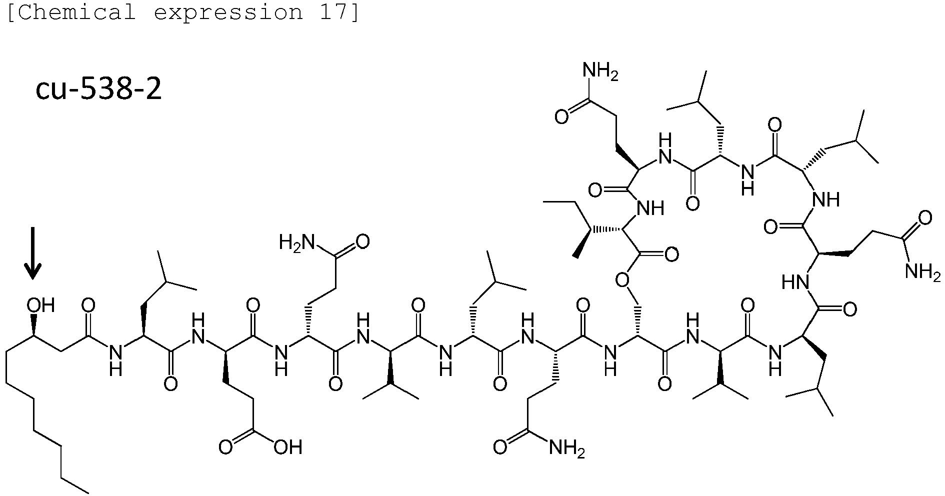

Specifically, these compounds were synthesized as described below.

300 mg (0.111 mmol) of Fmoc-NH-SAL Resin (0.37 mmol/g, Watanabe Chemical Industries Ltd.) was weighed out into a PP filtration column, and the resin was caused to swell for 40 minutes at room temperature in a dimethyl formamide (DMF) solution. After swelling, protective Fmoc (9-fluorenylmethoxycarbonyl) groups in the resin were removed by being reacted for 20 minutes at room temperature in a 20% (v/v) piperidine/DMF solution. Cleansing was performed ten times using DMF; Fmoc-D-Glu-Oallyl (0.333 mmol, 3 eq) was reacted in DMF for 30 minutes at room temperature in the presence of 1-hydroxy-7-azabenzotriazole (HOAt, 0.333 mmol, 3 eq), O-(7-aza-1H-benzotriazole-1-yl)-N,N,N',N'-tetramethyl uronium hexafluorophorphate (HATU, 0.333 mmol, 3 eq), and N,N-diisopropylamine (DIPEA, 0.333 mmol, 3 eq); and the amino acids were introduced into the resin. In order to condense subsequent amino acids, cleansing was performed ten times using DMF, and the protective Fmoc groups in the resin were removed by being reacted for 20 minutes at room temperature in a 20% (v/v) piperidine/DMF solution. Fmoc-Leu-OH (0.333 mmol, 3 eq), Fmoc-Leu-OH (0.333 mmol, 3 eq), Fmoc-D-Gln(Trt)-OH (0.333 mmol, 3 eq), Fmoc-D-Leu-OH (0.333 mmol, 3 eq), and Fmoc-D-Val-OH (0.333 mmol, 3 eq) were next introduced from the C terminal in sequence in the same manner as was the case with the Fmoc-D-Glu-Oallyl to elongate the peptide chain. The Fmoc groups in the resin were removed by being reacted for 20 minutes at room temperature in a 20% (v/v) piperidine/DMF solution. Cleansing was performed ten times using DMF, N(((9H-fluorene-9-yl)methoxy)carbonyl)-O-((2R)-2-(((allyloxy)carbonyl)amino)-3-methylpentanoyl)-D-serine (0.333 mmol, 3 eq) was reacted in dichloromethane (DCM) for 90 minutes at room temperature in the presence of N,N'-diisopropylcarbodiimide (DIPCI, 0.333 mmol, 3 eq) and 1-hydroxy-1H-benzotriazole hydrate (HOBt·H₂O, 0.333 mmol, 3 eq), and the amino acids were introduced into the resin. After this introduction, cleansing was performed five times using DMF, five times using methanol, and five times using diethyl ether, after which the resin was dried. A reaction was carried out on the dried resin in DCM for 180 minutes at room temperature in the presence of tetrakis(triphenylphosphine)palladium(0) (0.111 mmol, 1 eq) and phenylsilane (1.11 mmol, 10 eq), and allyl groups and allyloxycarbonyl (Alloc) groups in the resin were removed. In order to form rings in the resin, cleansing was performed five times using DCM, five times using a 0.5% (w/v) sodium N,N-diethyldithiocarbamate trihydrate/DMF solution, and five times using DMF, and rings were formed in DMF for 90 minutes at room temperature in the presence of N,N'-diisopropylcarbodiimide (DIPCI, 0.555 mmol, 5 eq) and 1-hydroxy-1H-benzotriazole hydrate (HOBt·H₂O, 0.555 mmol, 5 eq). In order to remove Fmoc groups in the resin, cleansing was performed ten times using DMF, and the Fmoc groups in the resin were removed by being reacted for five minutes at room temperature in a 20% (v/v) piperidine/DMF solution. Cleansing was performed ten times using DMF, Fmoc-Gln(Trt)-OH (0.333 mmol, 3 eq) was reacted in DMF for 90 minutes at room temperature in the presence of N,N'-diisopropylcarbodiimide (DIPCI, 0.333 mmol, 3 eq) and 1-hydroxy-1H-benzotriazole hydrate (HOBt·H₂O, 0.333 mmol, 3 eq), and the amino acids were introduced into the resin. In order to condense subsequent amino acids, cleaning was performed ten times using DMF, and Fmoc groups in the resin were removed by being reacted for 20 minutes at room temperature in a 20% (v/v) piperidine/DMF solution. Fmoc-D-Leu-OH (0.333 mmol, 3 eq), Fmoc-D-Val-OH (0.333 mmol, 3 eq), Fmoc-D-Gln(Trt)-OH (0.333 mmol, 3 eq), Fmoc-D-Glu(OtBu)-OH (0.333 mmol, 3 eq), Fmoc-Leu-OH (0.333 mmol, 3 eq), and (±)-3-(tert-butyldimethylsiloxane)decanoic acid (0.333 mmol, 3 eq) were next introduced from the C terminal in sequence in the same manner as was the case with the Fmoc-D-Glu-Oallyl; cleansing was performed five times using DMF, five times using methanol, and five times using diethyl ether; and then the resin was dried. For the purpose of removing various side-chain protective groups and removing resin, a reaction was carried out for three hours in 11.4 mL of trifluoroacetic acid (TFA) in the presence of water (0.6 mL). The reaction liquid was filtered, the filtered liquid was collected, TFA was volatilized in a nitrogen airflow and thereby distilled away, cleansing was performed by diethyl ether, and the solution was removed by decantation. The resultant residues were dried under reduced pressure, dissolved in a 70% (v/v) acetonitrile aqueous solution, and refined using high-performance liquid chromatography, whereby a white solid was obtained (cu538-1: 8.18 mg, yield: 8%; cu538-2: 7.75 mg, yield: 8%). Once the mass (MS) of the resultant compound and the NMR of the cu538-2 were analyzed, the following data were obtained.

### (MS data)

cu538-1: HRMS (ES+) calculated for C₈₄H₁₄₇N₁₈O₂₃ [M+H]⁺ 1776.0886 found 1776.0884
cu538-2: HRMS (ES+) calculated for C₈₄H₁₄₇N₁₈O₂₃ [M+H]⁺ 1776.0886 found 1776.0886

### (NMR data)

cu538-2: 1H NMR (600 MHz, MeOD): δ 4.62 (bs, 1H), 4.42-4.28 (m, 7H), 4.2 (dd, 1H, J=3.9, 11.2 Hz), 4.16-4.11 (m, 3H), 4.08-4.03 (m, 1H), 4.00 (t, 1H, J=6.7 Hz), 3.87 (bs, 1H), 3.78 (d, 2H, J=9.3 Hz), 2.52-2.50 (m, 3H), 2.42-2.30(m, 13H), 2.24-1.99 (m, 12H), 1.94-1.83 (m, 6H), 1.71-1.32 (m, 29H), 1.22-1.17 (m, 1H), 1.09 (d, 3H, J=6.5 Hz), 1.04 (d, 3H, J=6.6 Hz), 1.00-0.87 (m, 54H)
13C NMR (150 MHz, MeOD) : 175.5, 174.8, 174.7, 174.6, 174.6, 174.3, 173.5, 173.2, 171.9, 69.8, 64.9, 64.3, 58.7, 58.6, 57.3, 57.1, 56.6, 55.7, 55.6, 54.9, 54.8, 54.6, 54.5, 54.3, 53.9, 53.8, 53.8, 44.4, 41.6, 41.5, 40.5, 40.5, 38.4, 37.4, 33.2, 33.0, 32.9, 32.7, 32.6, 31.1, 31.0, 30.7, 30.6, 30.5, 30.4, 27.7, 27.4, 27.4, 26.9, 26.6, 26.1, 26.0, 25.9, 25.8, 25.8, 25.6, 23.7, 23.7, 23.6, 23.5, 22.6, 22.5, 22.1, 21.6, 21.1, 20.9, 20.7, 19.7, 19.4, 16.2, 14.4, 11.3

### [Preparation example 2] (Solid-phase synthesis of NY-38)

The NY-38 illustrated below was synthesized by Fmoc solid-phase synthesis in the same manner as preparation example 1.

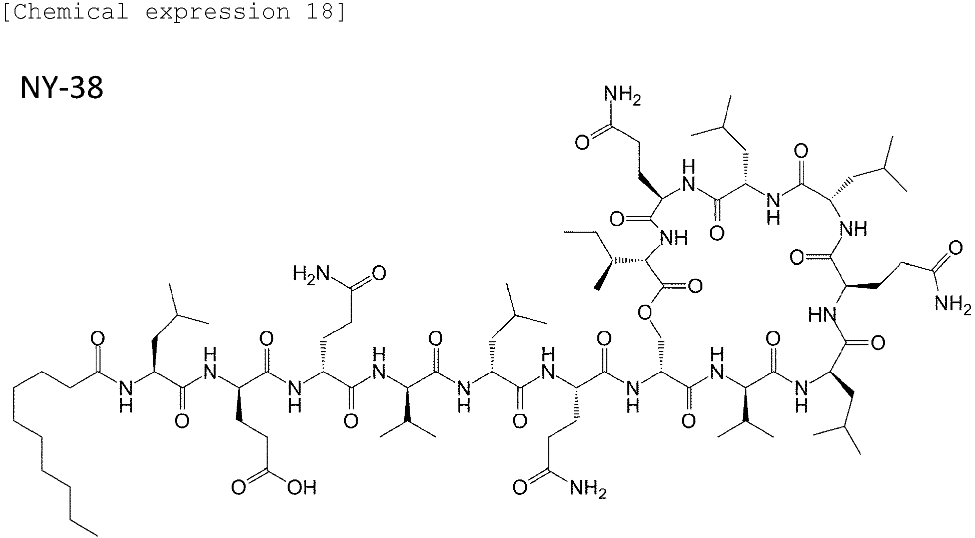

Specifically, this compound was synthesized as follows.

100 mg (0.037 mmol) of Fmoc-NH-SAL Resin (0.37 mmol/g, Watanabe Chemical Industries Ltd.) was weighed out into a PP filtration column, and the resin was caused to swell for 40 minutes at room temperature in a dimethyl formamide (DMF) solution. Synthesis involved using the same method as for cu538-1 and -2 to carry out elongation to the leucine of the first residue of the N terminal. After removal of Fmoc groups, decanoic acid (0.111 mmol, 3 eq) was introduced in the presence of N,N'-diisopropylcarbodiimide (DIPCI, 0.111 mmol, 3 eq) and 1-hydroxy-1H-benzotriazole hydrate (HOBt·H₂O, 0.333 mmol, 3 eq), and cleansing was performed five times using DMF, then five times using methanol and five times using diethyl ether, after which the resin was dried. For the purpose of removing various side-chain protective groups and removing resin, a reaction was carried out for three hours in 3.8 mL of trifluoroacetic acid (TFA) in the presence of water (0.2 mL). The reaction liquid was filtered, the filtered liquid was collected, TFA was volatilized in a nitrogen airflow and thereby distilled away, cleansing was performed by diethyl ether, and the solution was removed by decantation. The resultant residues were dried under reduced pressure, dissolved in a 70% (v/v) acetonitrile aqueous solution, and refined using high-performance liquid chromatography, whereby a white solid was obtained (2.6 mg, yield: 4%). Once the mass (MS) of the resultant compound was analyzed, the following data were obtained.

### (MS data)

NY-38: HRMS (ES+) calculated for C₈₄H₁₄₇N₁₈O₂₂ [M+H]⁺ 1760.0937 found 1760.0916

### [Preparation example 3] (Solid-phase synthesis of cu592)

The cu592 illustrated below was synthesized by Fmoc solid-phase synthesis in the same manner as preparation example 1.

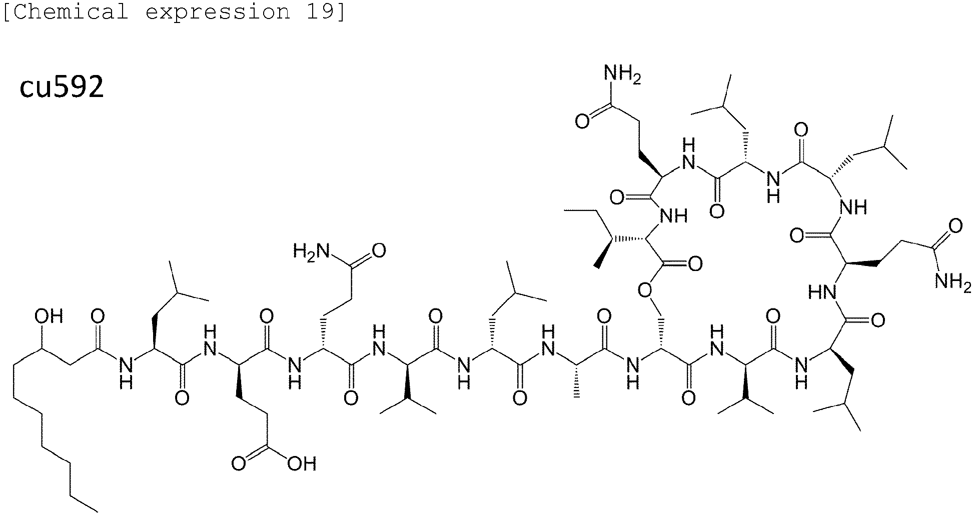

Specifically, this compound was synthesized as follows.

50 mg (0.0185 mmol) of Fmoc-NH-SAL Resin (0.37 mmol/g, Watanabe Chemical Industries Ltd.) was weighed out into a PP filtration column, and the resin was caused to swell for 40 minutes at room temperature in a dimethyl formamide (DMF) solution. Synthesis involved using the same method as for cu538-1 and -2 to introduce Fmoc-D-Glu-Oallyl (0.0555 mmol), Fmoc-Leu-OH (0.0555 mmol, 3 eq), Fmoc-Leu-OH (0.0555 mmol, 3 eq), Fmoc-D-Gln(Trt)-OH (0.0555 mmol, 3 eq), Fmoc-D-Leu-OH (0.0555 mmol, 3 eq), Fmoc-D-Val-OH (0.0555 mmol, 3 eq), and N(((9H-fluorene-9-yl)methoxy)carbonyl)-O-((2R)-2-(((allyloxy)carbonyl)amino)-3-methylpentanoyl)-D-serine (0.0555 mmol, 3 eq) from the C terminal in sequence. After this introduction, a ring-forming reaction was performed using the same method as for cu538-1 and -2. Fmoc groups in the resin were then deprotected, and Fmoc-Ala-OH (0.0555 mmol, 3 eq), Fmoc-D-Leu-OH (0.0555 mmol, 3 eq), Fmoc-D-Val-OH (0.0555 mmol, 3 eq), Fmoc-D-Gln(Trt)-OH (0.0555 mmol, 3 eq), Fmod-D-Glu(OtBu)-OH (0.0555 mmol, 3 eq), Fmoc-Leu-OH (0.0555 mmol, 3 eq), and (+)-3-(tert-butyldimethylsiloxy)decanoic acid (0.0555 mmol, 3 eq) were introduced. After resin was removed using the same method as for cu538-1 and -2, refining was carried out using high-performance liquid chromatography, whereby a white solid was obtained (3.18 mg, yield: 10%). Once the mass (MS) of the resultant compound was analyzed, the following data were obtained.

### (MS data)

cu592: HRMS (ES+) calculated for C₈₂H₁₄₄N₁₇O₂₂ [M+H]⁺ 1719.0672 found 1719.0659

### [Preparation example 4] (Solid-phase synthesis of cu605)

The cu605 illustrated below was synthesized by Fmoc solid-phase synthesis in the same manner as preparation example 1.

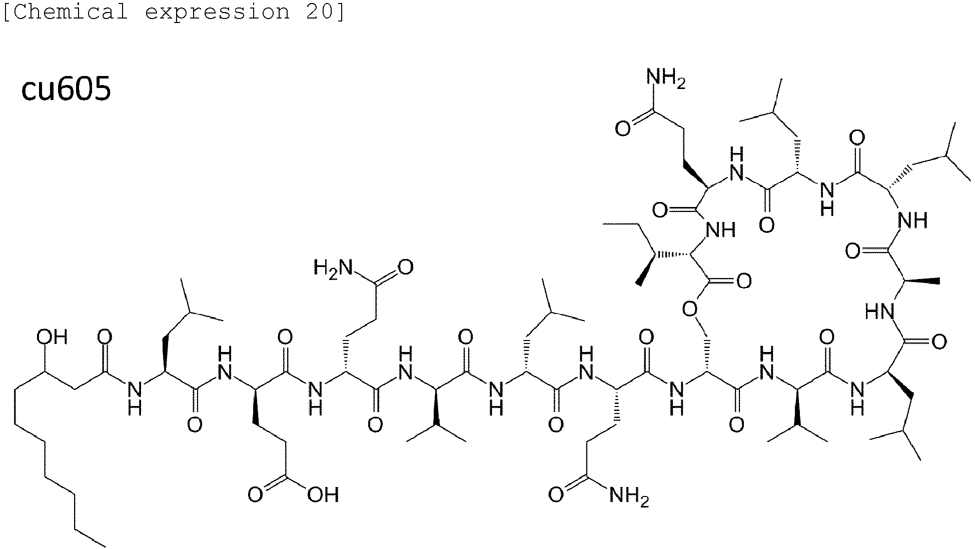

Specifically, this compound was synthesized as follows.

100 mg (0.037 mmol) of Fmoc-NH-SAL Resin (0.37 mmol/g, Watanabe Chemical Industries Ltd.) was weighed out into a PP filtration column, and the resin was caused to swell for 40 minutes at room temperature in a dimethyl formamide (DMF) solution. Synthesis involved using the same method as for cu538-1 and -2 to introduce Fmoc-D-Glu-Oallyl (0.111 mmol), Fmoc-Leu-OH (0.111 mmol, 3 eq), Fmoc-Leu-OH (0.111 mmol, 3 eq), Fmoc-D-Ala-OH (0.111 mmol, 3 eq), Fmoc-D-Leu-OH (0.111 mmol, 3 eq), Fmoc-D-Val-OH (0.111 mmol, 3 eq), and N(((9H-fluorene-9-yl)methoxy)carbonyl)-O-((2R)-2-(((allyloxyl)carbonyl)amino)-3-methylpentanoyl)-D-serine (0.111 mmol, 3 eq) from the C terminal in sequence. After this introduction, a ring-forming reaction was performed using the same method as for cu538-1 and -2. Fmoc groups in the resin were then deprotected, and Fmoc-Ala-OH (0.111 mmol, 3 eq), Fmoc-D-Leu-OH (0.111 mmol, 3 eq), Fmoc-D-Val-OH (0.111 mmol, 3 eq), Fmoc-D-Gln(Trt)-OH (0.111 mmol, 3 eq), Fmoc-D-Glu(OtBu)-OH (0.111 mmol, 3 eq), Fmoc-Leu-OH (0.111 mmol, 3 eq), and (+)-3-(tert-butyldimethylsiloxy)decanoic acid (0.111 mmol, 3 eq) were introduced. After resin was removed using the same method as for cu538-1 and -2, refining was carried out using high-performance liquid chromatography, whereby a white solid was obtained (1.53 mg, yield: 2%). Once the mass (MS) of the resultant compound was analyzed, the following data were obtained.

### (MS data)

cu605: HRMS (ES+) calculated for C₈₂H₁₄₄N₁₇O₂₂ [M+H]⁺ 1719.0672 found 1719.0677

### [Preparation example 5] (Solid-phase synthesis of cu619)

The cu619 illustrated below was synthesized by Fmoc solid-phase synthesis in the same manner as preparation example 1.

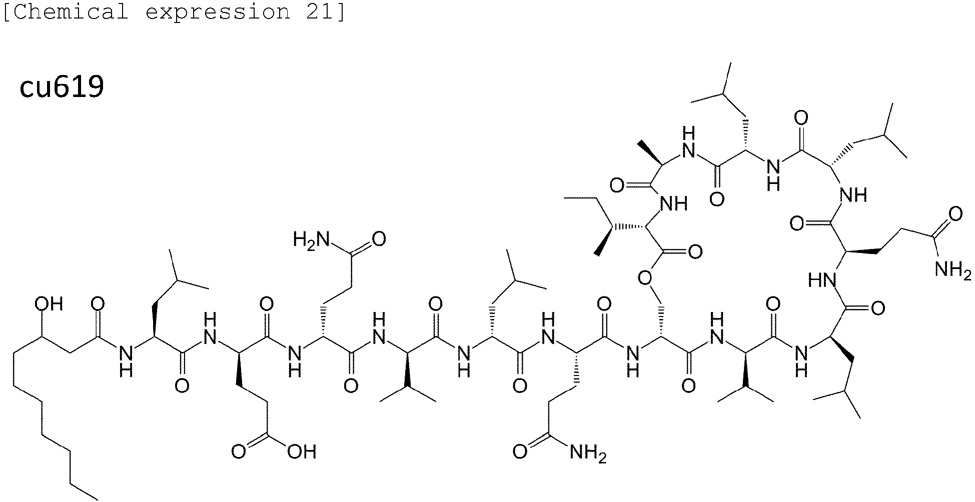

Specifically, this compound was synthesized as follows.

100 mg (0.037 mmol) of Fmoc-NH-SAL Resin (0.37 mmol/g, Watanabe Chemical Industries Ltd.) was weighed out into a PP filtration column, and the resin was caused to swell for 40 minutes at room temperature in a dimethyl formamide (DMF) solution. Synthesis involved using the same method as for cu538-1 and -2 to introduce Fmoc-D-Glu-Oallyl (0.111 mmol), Fmoc-D-Leu-OH (0.111 mmol, 3 eq), Fmoc-D-Val-OH (0.111 mmol, 3 eq), Fmoc-D-Ser-OH (0.111 mmol, 3 eq), Fmoc-Gln(Trt)-OH (0.111 mmol, 3 eq), Fmoc-D-Leu-OH (0.111 mmol, 3 eq), Fmoc-D-Val-OH (0.111 mmol, 3 eq), Fmoc-D-Gln(Trt)-OH (0.111 mmol, 3 eq), Fmoc-D-Glu(OtBu)-OH (0.111 mmol, 3 eq), Fmoc-Leu-OH (0.111 mmol, 3 eq), and (+)-3-(tert-butyldimethylsiloxy)decanoic acid (0.111 mmol, 3 eq) from the C terminal in sequence, and cleansing was performed five times using DMF, then five times using methanol and five times using diethyl ether, after which the resin was dried under reduced pressure. Fmoc-Ile-OH (0.111 mmol, 3 eq) was reacted with the dried resin using anhydrous DMF for 120 minutes at room temperature in the presence of N,N'-diisopropylcarbodiimide (DIPCI, 0.185 mmol, 5 eq), pyridine (0.185 mmol, 5 eq), and N,N-dimethyl-4-aminopyridine (0.185 mmol, 5 eq). This operation was performed twice, and the amino acids were introduced into the resin. Fmoc groups in the isoleucine residues were deprotected, and then Fmoc-D-Ala-OH (0.111 mmol, 3 eq), Fmoc-Leu-OH (0.111 mmol, 3 eq), and Fmoc-Leu-OH (0.111 mmol, 3 eq) were introduced. After this introduction, cleansing was performed five times using DMF, five times using methanol, and five times using diethyl ether, and then the resin was dried. A reaction was performed on the dried resin in DCM for 180 minutes at room temperature in the presence of tetrakis(triphenylphosphine)palladium(0) (0.111 mmol, 1 eq) and phenylsilane (1.11 mmol, 10 eq), and allyl groups in the resin were removed. In order to form rings in the resin, cleansing was performed five times using DCM, five times using a 0.5% (w/v) sodium N,N-diethyldithiocarbamate trihydrate/DMF solution, and five times using DMF, and Fmoc groups were deprotected, after which rings were formed. After resin was removed using the same method as for cu538-1 and -2, refining was carried out using high-performance liquid chromatography, whereby a white solid was obtained (11 mg, yield: 17%). Once the mass (MS) of the resultant compound was analyzed, the following data were obtained.

### (MS data)

cu619: HRMS (ES+) calculated for C₈₂H₁₄₃N₁₇O₂₂Na [M+Na]⁺ 1741.0491 found 1741.0494

### [Preparation example 6] (Solid-phase synthesis of L-Leu1L-Ala)

The L-Leu1L-Ala illustrated below was synthesized by Fmoc solid-phase synthesis in the same manner as preparation example 1. 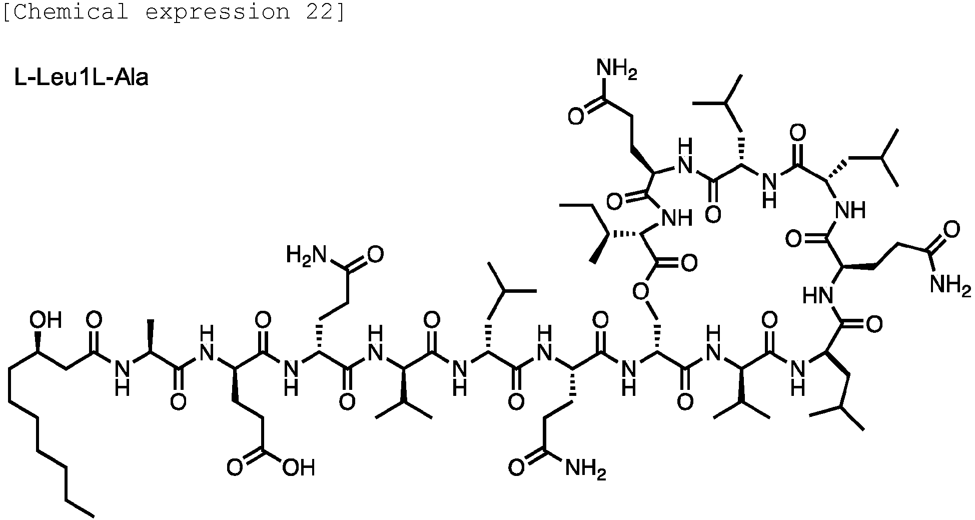 Yield: 10%

### (MS data)

L-Leu1L-Ala: HRMS (ES+) calculated for C₈₁H₁₄₃N₁₈O₂₃ [M+H]⁺ 1734.0147 found 1734.0393

### [Preparation example 7] (Solid-phase synthesis of D-Glu2D-Ala)

The D-Glu2D-Ala illustrated below was synthesized by Fmoc solid-phase synthesis in the same manner as preparation example 1. 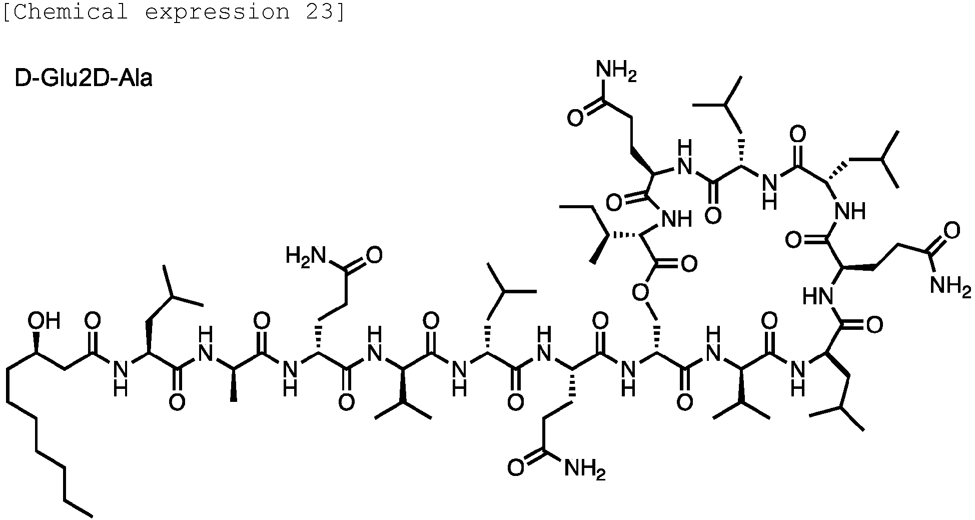 Yield: 8%

### (MS data)

D-Glu2D-Ala: HRMS(ES+) calculated for C₈₂H₁₄₅N₁₈O₂₁ [M+H]⁺ 1718.0832 found 1718.0809

### [Preparation example 8] (Solid-phase synthesis of D-Gln3D-Ala)

The D-Gln3D-Ala illustrated below was synthesized by Fmoc solid-phase synthesis in the same manner as preparation example 1. 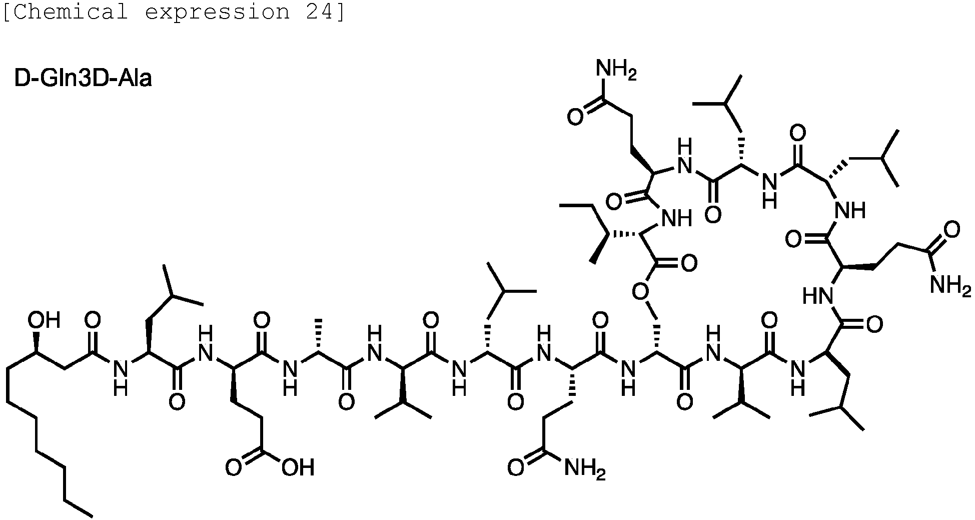 Yield: 10%

### (MS data)

D-Gln3D-Ala: HRMS(ES+) calculated for C₈₂H₁₄₄N₁₇O₂₂ [M+H]⁺ 1719.0672 found 1719.0649

### [Preparation example 9] (Solid-phase synthesis of D-Val4D-Ala)

The D-Val4D-Ala illustrated below was synthesized by Fmoc solid-phase synthesis in the same manner as preparation example 1. 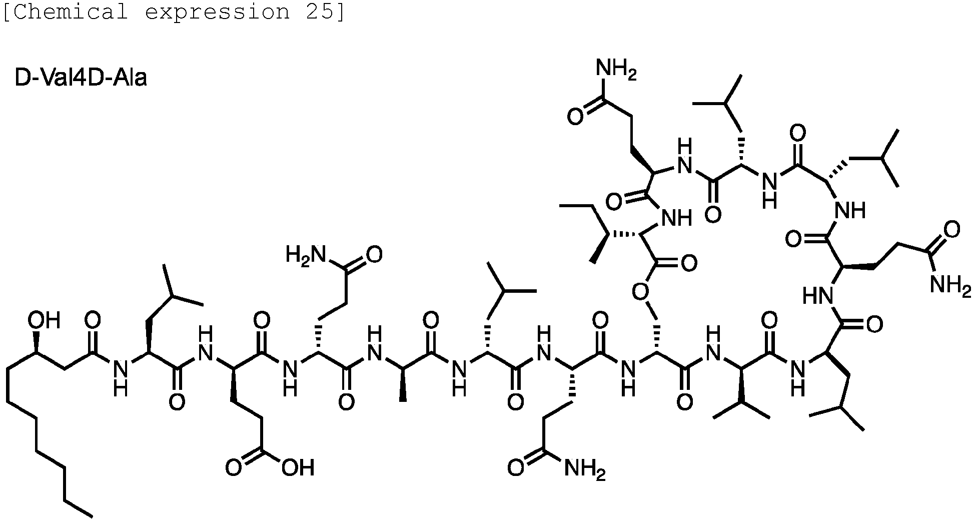 Yield: 9%

### (MS data)

D-Val4D-Ala: HRMS(ES+) calculated for C₈₂H₁₄₃N₁₈O₂₃ [M+H]⁺ 1748.0573 found 1748.0537

### [Preparation example 10] (Solid-phase synthesis of D-Leu5D-Ala)

The D-Leu5D-Ala illustrated below was synthesized by Fmoc solid-phase synthesis in the same manner as preparation example 1. 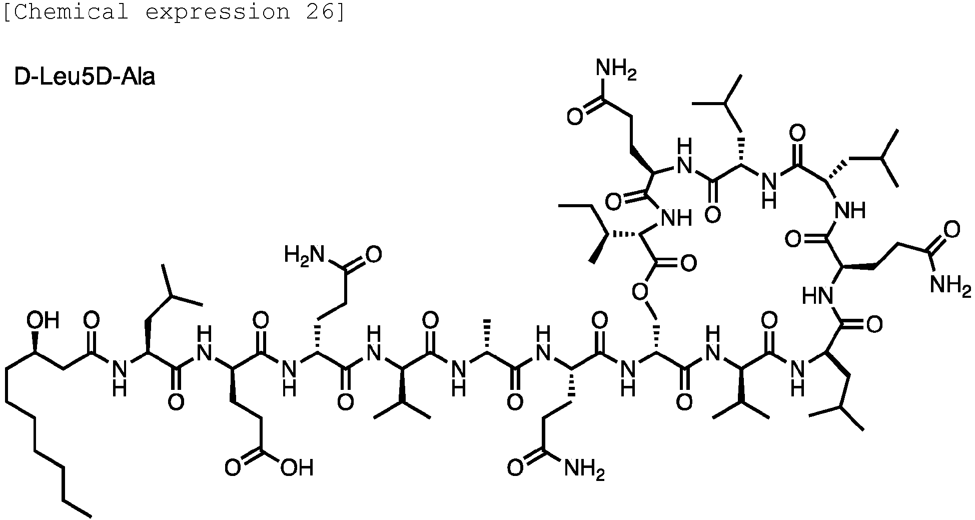 Yield: 9%

### (MS data)

D-Leu5D-Ala: HRMS(ES+) calculated for C₈₁H₁₄₁N₁₈O₂₃ [M+H]⁺ 1734.0417 found 1734.0397

### [Preparation example 11] (Solid-phase synthesis of D-Val8D-Ala)

The D-Val8D-Ala illustrated below was synthesized by Fmoc solid-phase synthesis in the same manner as preparation example 1. 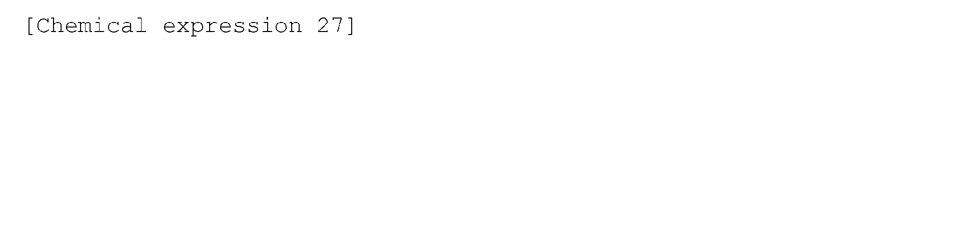 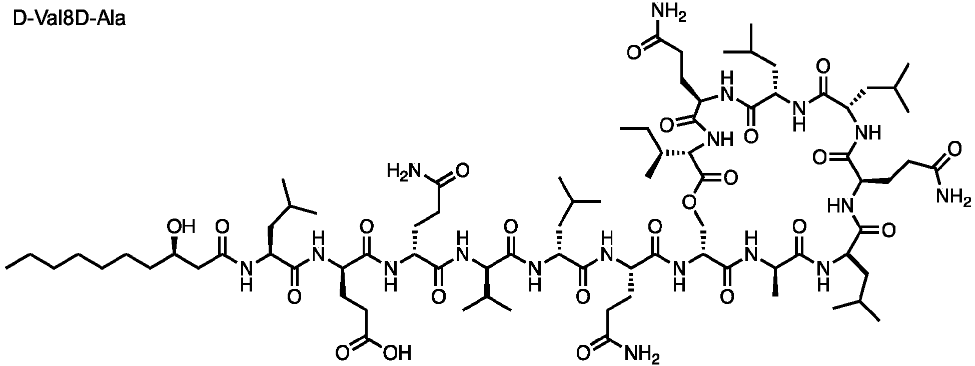 Yield: 1%

### (MS data)

D-Val8D-Ala: HRMS(ES+) calculated for C₈₂H₁₄₂N₁₈O₂₃Na [M+Na]⁺ 1770.0393 found 1770.0393

### [Preparation example 12] (Solid-phase synthesis of D-Leu9D-Ala)

The D-Leu9D-Ala illustrated below was synthesized by Fmoc solid-phase synthesis in the same manner as preparation example 1. 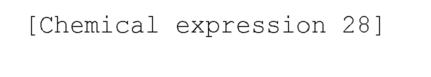 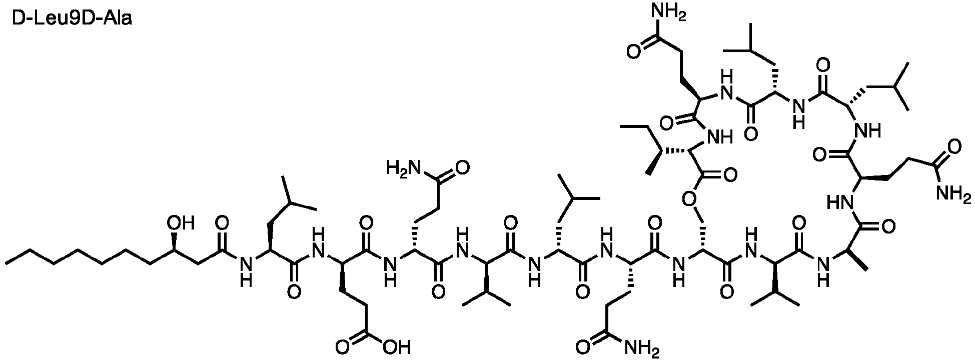 Yield: 3%

### (MS data)

D-Leu9D-Ala: HRMS(ES+) calculated for C₈₁H₁₄₀N₁₈O₂₃Na [M+Na]⁺ 1756.0236 found 1756.0238

### [Preparation example 13] (Solid-phase synthesis of L-Leu11L-Ala)

The L-Leu11L-Ala illustrated below was synthesized by Fmoc solid-phase synthesis in the same manner as preparation example 1. 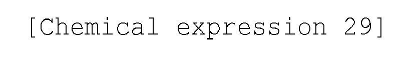 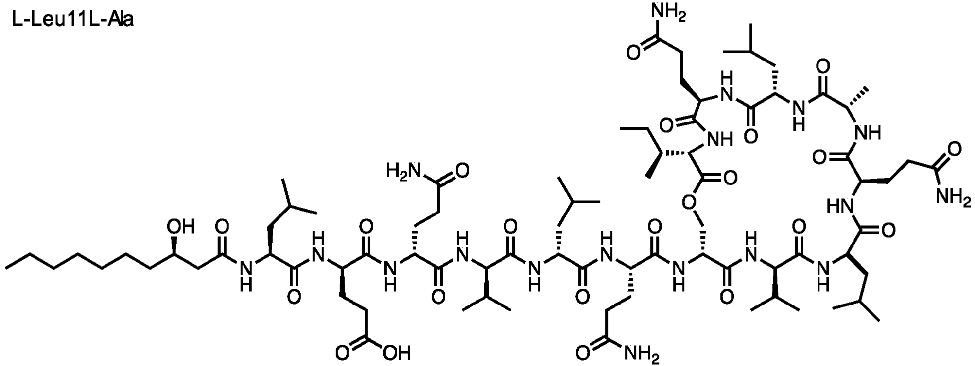 Yield: 5%

### (MS data)

L-Leu11L-Ala: HRMS(ES+) calculated for C₈₁H₁₄₃N₁₈O₂₃ [M+H]⁺ 1734.0417 found 1734.0393

### [Preparation example 14] (Solid-phase synthesis of L-Leu12L-Ala)

The L-Leu12L-Ala illustrated below was synthesized by Fmoc solid-phase synthesis in the same manner as preparation example 1. 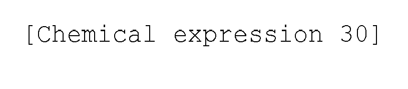 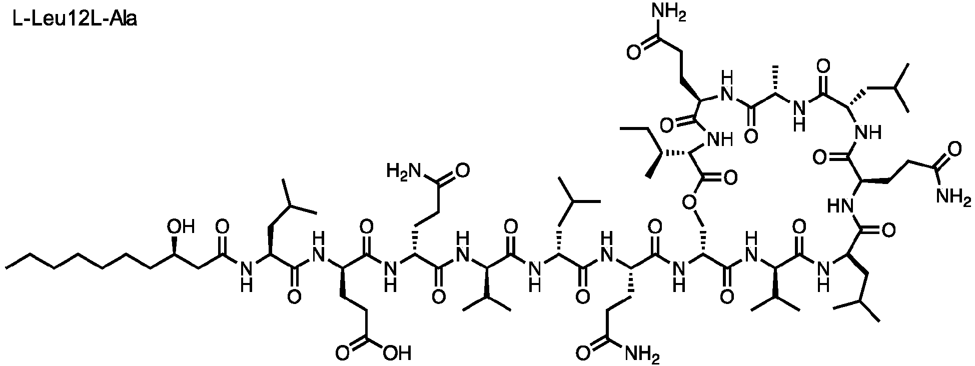 Yield: 1%

### (MS data)

L-Leu12L-Ala: HRMS(ES+) calculated for C₈₁H₁₄₃N₁₈O₂₃ [M+H]⁺ 1734.0417 found 1734.0393

### [Preparation example 15] (Solid-phase synthesis of L-Ile14L-Ala)

The L-Ile14L-Ala illustrated below was synthesized by Fmoc solid-phase synthesis in the same manner as preparation example 1.

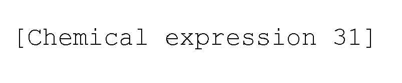

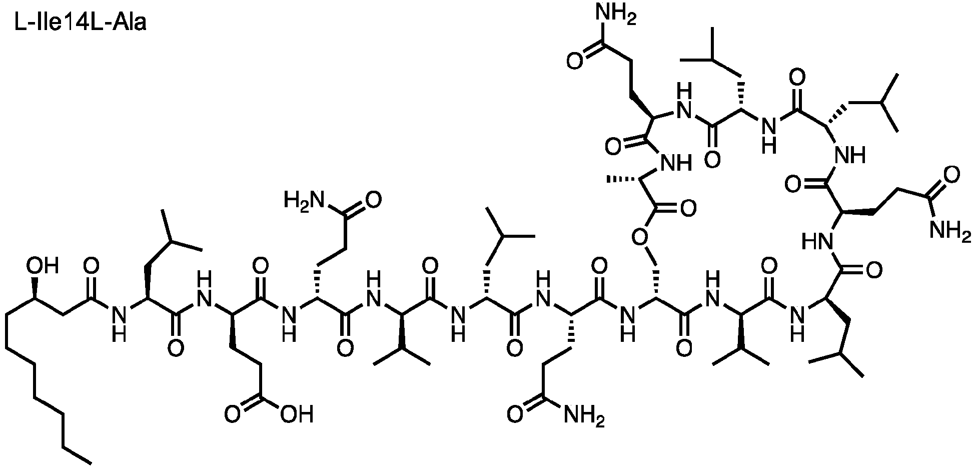

Yield: 26%

### (MS data)

L-Ile14L-Ala: HRMS(ES+) calculated for C₈₁H₁₄₁N₁₈O₂₃ [M+H]⁺ 1734.0417 found 1734.0420

### [Preparation example 16] (Solid-phase synthesis of cu632)

The cu632 illustrated below was synthesized by Fmoc solid-phase synthesis in the same manner as preparation example 1.

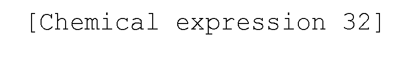

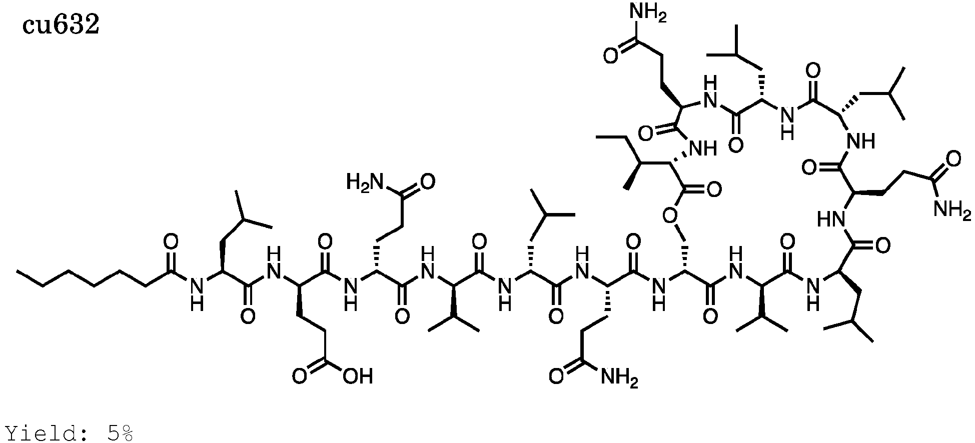

### (MS data)

cu632: HRMS (ES+) calculated for C₈₁H₁₄₁N₁₈O₂₂ [M+H]⁺ 1718.0468 found 1718.0468

### [Preparation example 17] (Solid-phase synthesis of cu633)

The cu633 illustrated below was synthesized by Fmoc solid-phase synthesis in the same manner as preparation example 1. 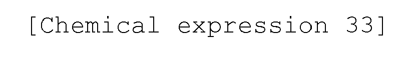 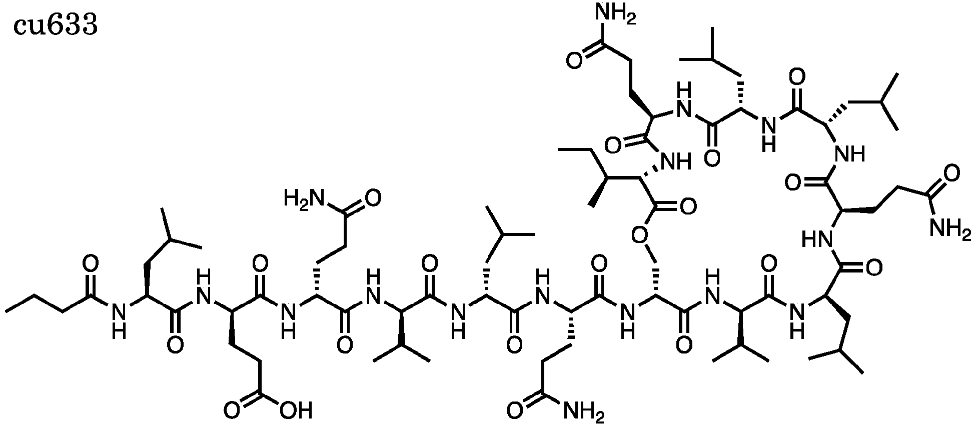 Yield: 5%

### (MS data)

cu633: HRMS (ES+) calculated for C₈₁H₁₄₁N₁₈O₂₃ [M+H]⁺ 1675.9998 found 1676.0007

### [Preparation example 18] (Solid-phase synthesis of L-Leu1D-Leu)

The L-Leu1D-Leu illustrated below was synthesized by Fmoc solid-phase synthesis in the same manner as preparation example 1. 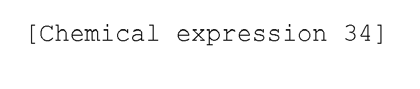 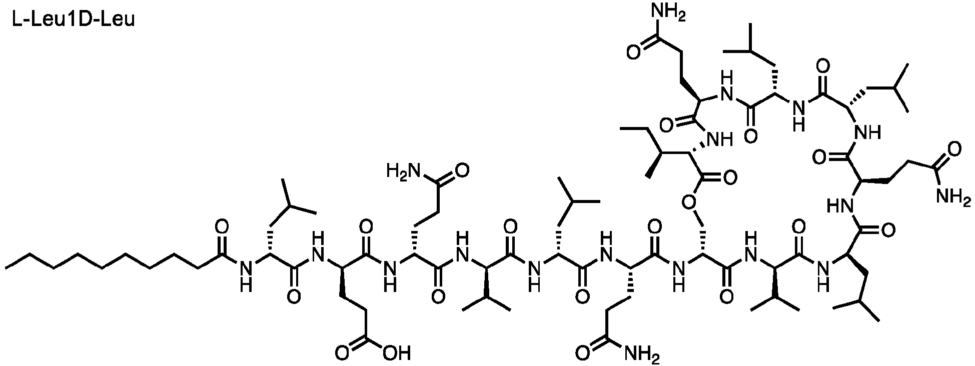 Yield: 12%

### (MS data)

L-Leu1D-Leu: HRMS(ES+) calculated for C₈₄H₁₄₇N₁₈O₂₂ [M+H]⁺ 1760.0937 found 1760.0928

### [Preparation example 19] (Solid-phase synthesis of D-Glu2L-Glu)

The D-Glu2L-Glu illustrated below was synthesized by Fmoc solid-phase synthesis in the same manner as preparation example 1. 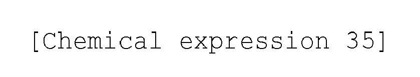 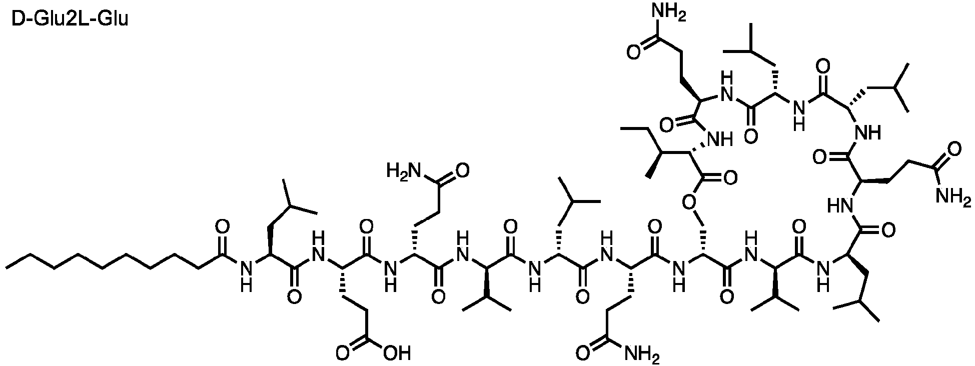 Yield: 16%

### (MS data)

D-Glu2L-Glu: HRMS(ES+) calculated for C₈₄H₁₄₇N₁₈O₂₂ [M+H]⁺ 1760.0937 found 1760.0923

### [Preparation example 20] (Solid-phase synthesis of D-Gln3L-Gln)

The D-Gln3L-Gln illustrated below was synthesized by Fmoc solid-phase synthesis in the same manner as preparation example 1. 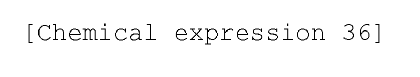 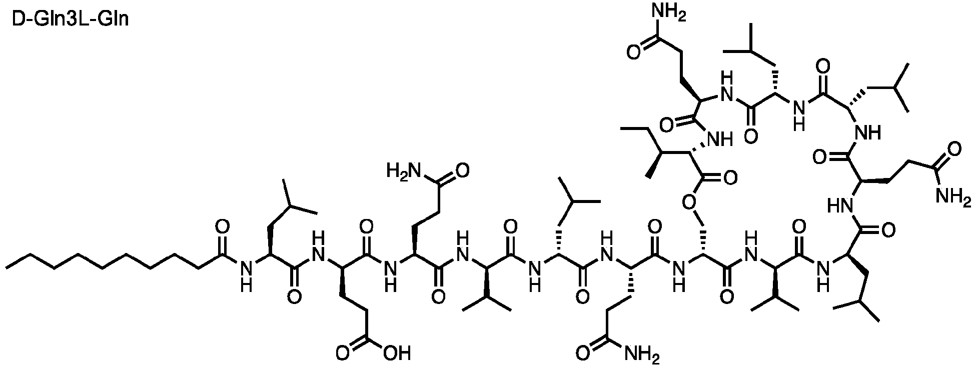 Yield: 11%

### (MS data)

D-Gln3L-Gln: HRMS(ES+) calculated for C₈₄H₁₄₇N₁₈O₂₂ [M+H]⁺ 1760.0937 found 1760.0920

### [Preparation example 21] (Solid-phase synthesis of D-Val4L-Val)

The D-Val4L-Val illustrated below was synthesized by Fmoc solid-phase synthesis in the same manner as preparation example 1. 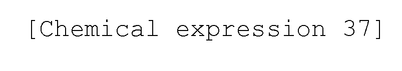 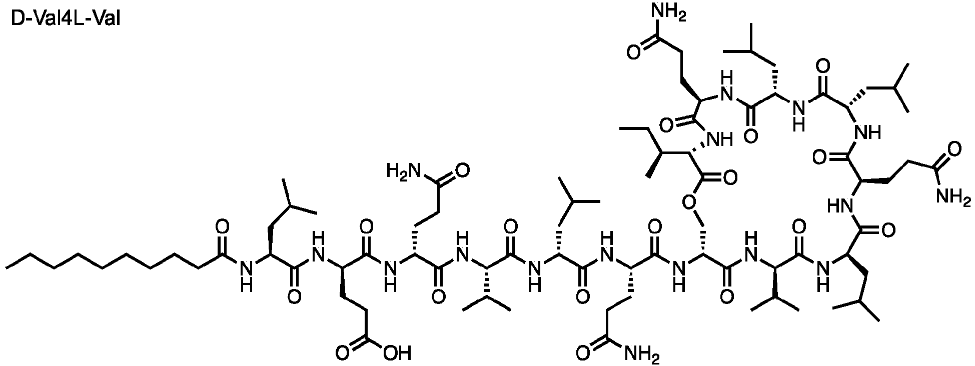 Yield: 11%

### (MS data)

D-Val4L-Val: HRMS(ES+) calculated for C₈₄H₁₄₇N₁₈O₂₂ [M+H]⁺ 1760.0937 found 1760.0920

### [Preparation example 22] (Solid-phase synthesis of D-Leu5L-Leu)

The D-Leu5L-Leu illustrated below was synthesized by Fmoc solid-phase synthesis in the same manner as preparation example 1. 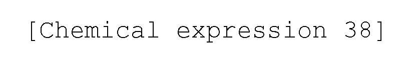 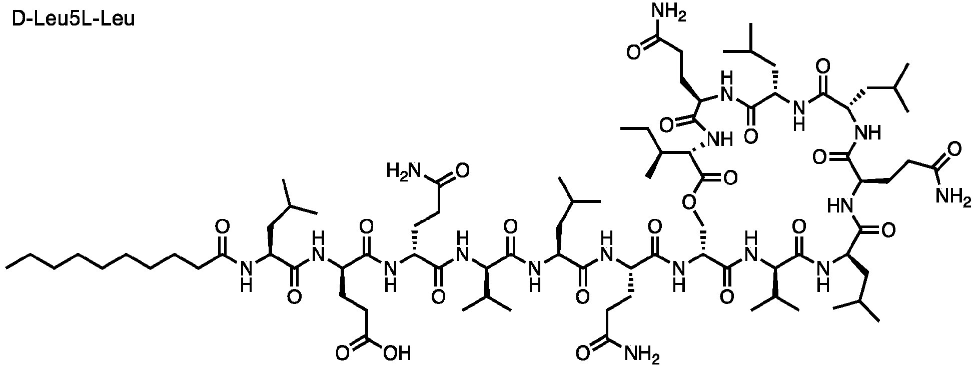 Yield: 5%

### (MS data)

D-Leu5L-Leu: HRMS(ES+) calculated for C₈₄H₁₄₇N₁₈O₂₂ [M+H]⁺ 1760.0937 found 1760.0920

### [Preparation example 23] (Solid-phase synthesis of L-Gln6D-Gln)

The L-Gln6D-Gln illustrated below was synthesized by Fmoc solid-phase synthesis in the same manner as preparation example 1. 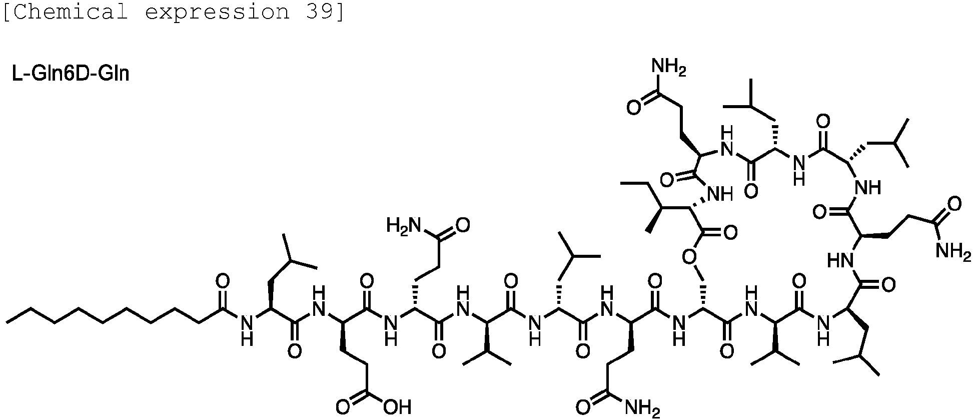 Yield: 21%

### (MS data)

L-Gln6D-Gln: HRMS(ES+) calculated for C₈₄H₁₄₇N₁₈O₂₂ [M+H]⁺ 1760.0937 found 1760.0923

### [Preparation example 24] (Solid-phase synthesis of D-Val8L-Val)

The D-Val8L-Val illustrated below was synthesized by Fmoc solid-phase synthesis in the same manner as preparation example 1. 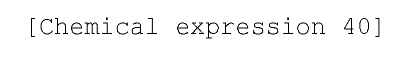 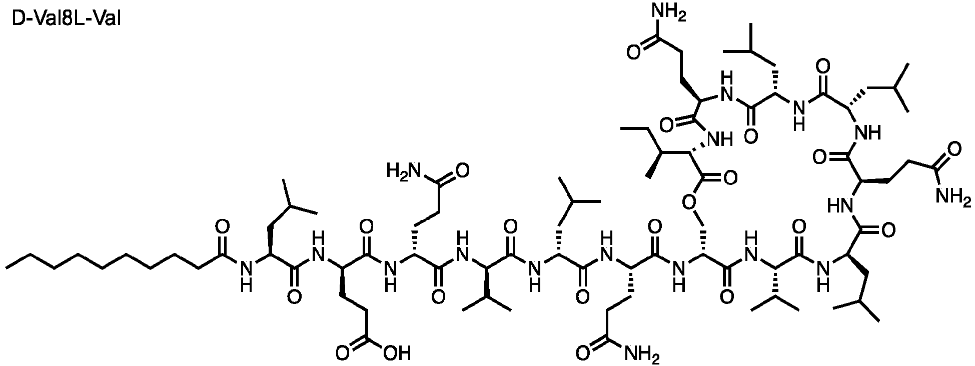 Yield: 9%

### (MS data)

D-Val8L-Val: HRMS(ES+) calculated for C₈₄H₁₄₆N₁₈O₂₂Na [M+Na]⁺ 1782.0757 found 1782.0748

### [Preparation example 25] (Solid-phase synthesis of D-Leu9L-Leu)

The D-Leu9L-Leu illustrated below was synthesized by Fmoc solid-phase synthesis in the same manner as preparation example 1. 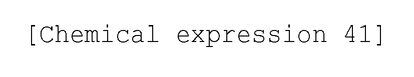 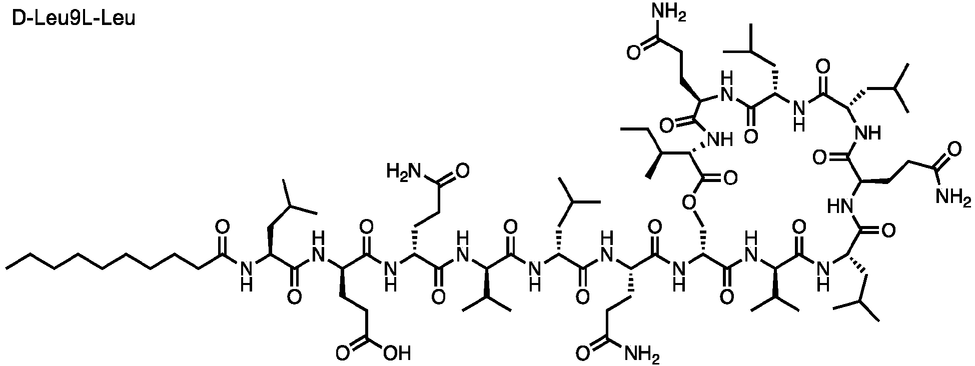 Yield: 4%

### (MS data)

D-Leu9L-Leu: HRMS(ES+) calculated for C₈₄H₁₄₇N₁₈O₂₂ [M+H]⁺ 1760.0937 found 1760.0938

### [Preparation example 26] (Solid-phase synthesis of D-Gln10L-Gln)

The D-Gln10L-Gln illustrated below was synthesized by Fmoc solid-phase synthesis in the same manner as preparation example 1. 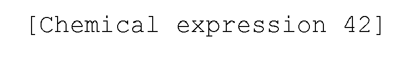 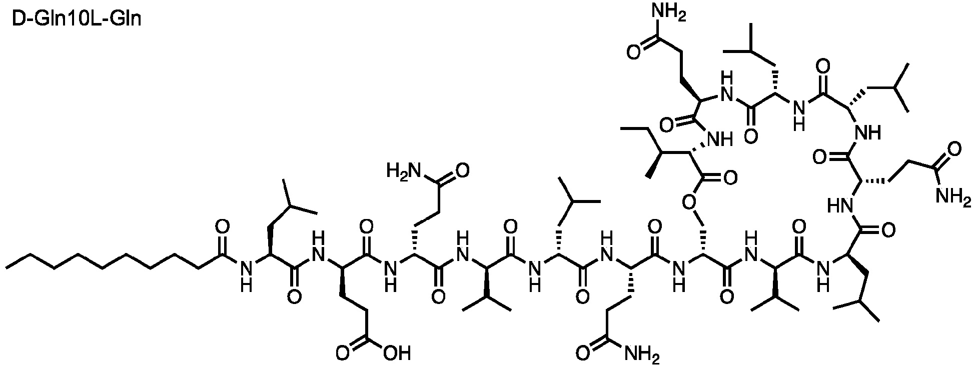 Yield: 12%

### (MS data)

D-Gln10L-Gln: HRMS(ES+) calculated for C₈₄H₁₄₆N₁₈O₂₂Na [M+Na]⁺ 1782.0757 found 1782.0753

### [Preparation example 27] (Solid-phase synthesis of L-Leu11D-Leu)

The L-Leu11D-Leu illustrated below was synthesized by Fmoc solid-phase synthesis in the same manner as preparation example 1. 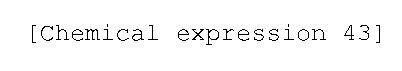 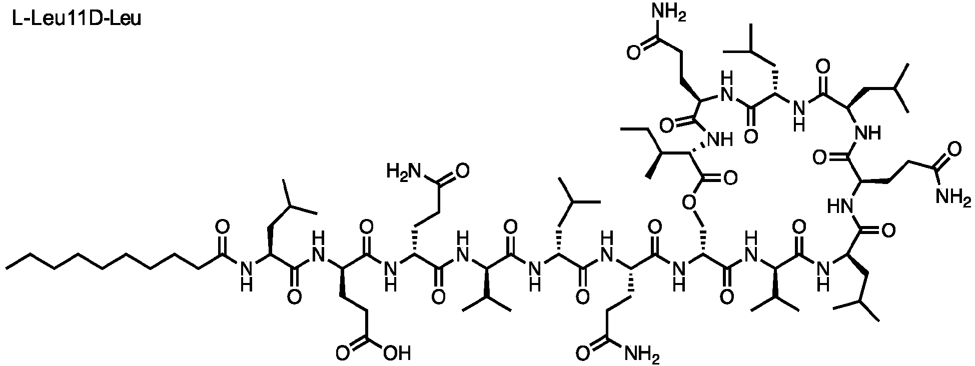 Yield: 6%

### (MS data)

L-Leu11D-Leu: HRMS(ES+) calculated for C₈₄H₁₄₇N₁₈O₂₂ [M+H]⁺ 1782.0757 found 1782.0753

### [Preparation example 28] (Solid-phase synthesis of L-Leu12D-Leu)

The L-Leu12D-Leu illustrated below was synthesized by Fmoc solid-phase synthesis in the same manner as preparation example 1. 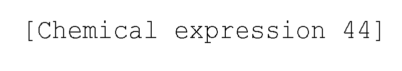 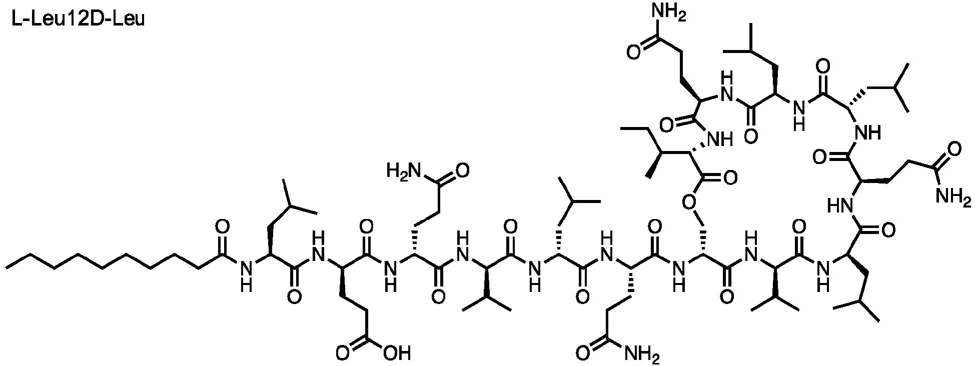 Yield: 14%

### (MS data)

L-Leu12D-Leu: HRMS(ES+) calculated for C₈₄H₁₄₇N₁₈O₂₂ [M+H]⁺ 1760.0937 found 1760.0938

### [Preparation example 29] (Solid-phase synthesis of D-Gln13L-Gln)

The D-Gln13L-Gln illustrated below was synthesized by Fmoc solid-phase synthesis in the same manner as preparation example 1. 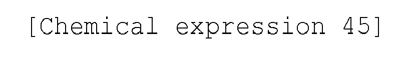 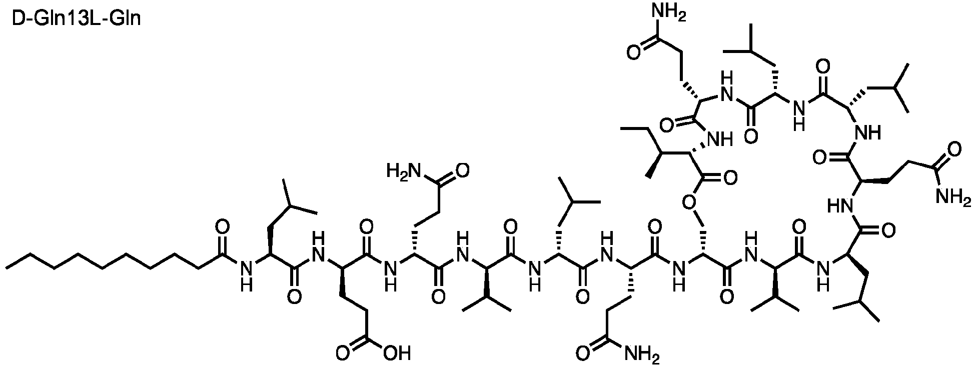 Yield: 7%

### (MS data)

D-Gln13L-Gln: HRMS(ES+) calculated for C₈₄H₁₄₇N₁₈O₂₂ [M+H]⁺ 1760.0937 found 1760.0928

### [Preparation example 30] (Solid-phase synthesis of L-Ile14D-Ile)

The L-Ile14D-Ile illustrated below was synthesized by Fmoc solid-phase synthesis in the same manner as preparation example 1. 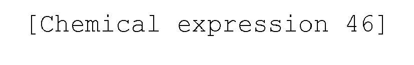 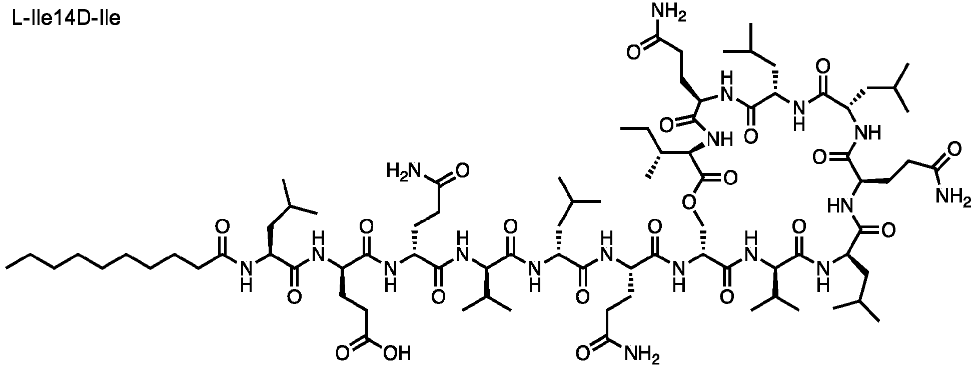 Yield: 16%

### (MS data)

L-Ile14D-Ile: HRMS(ES+) calculated for C₈₄H₁₄₇N₁₈O₂₂ [M+H]⁺ 1760.0937 found 1760.0942

### <Test example 1>

MDCK II cells derived from dog kidney epithelial cells were used to create a model of an epithelial cell layer in which a transwell was used, and a test to evaluate permeation by substances was conducted. Specifically, MDCK II cells in a logarithmic growth phase were sowed in a transwell (diameter: 6.5 mm, collagen coat, pore size: 0.4 µm) (trade name "Corning Transwell 3495," made by Corning Co., Ltd.) at a cell count of 3.4 to 4.0×10⁴ cells, a 24-hole well plate was used as basal-side wells, and 600 µL of a cell growth medium was introduced into each of apical-side wells and the basal-side wells. The medium was replaced daily to form a layer, and three days after sowing the medium was replaced with a Hank's balanced salt solution (HBSS) for measurement. After incubation for one hour at 37°C, layer formation was confirmed by measuring the transepithelial electrical resistance (TEER) using a resistance value measurement system (trade name "Millicell-ERS," made by Millipore Corp.). After layer formation was confirmed, the apical-side medium was replaced by 100 µL of HBSS to which a marker substance (FD4: fluorescent indicator dextran) (made by Sigma KK) having a molecular weight of 4000 was added so as to reach a concentration of 1.0 w/v%, and test substances were added so as to reach the final concentrations indicated below. The transwell was shifted, at intervals of 30 minutes until five hours after addition of the marker substance, to the basal-side wells into which the new HBSS entered, and the HBSS in the remaining basal-side wells (referred to as a "receiver solution" below) was retrieved 50 µL at a time per well in a 96-hole well plate. The fluorescence intensity of the retrieved HBSS at an excitation wavelength of 485 nm and a fluorescence wavelength of 528 nm was measured using a fluorescent plate reader (trade name "Wallac 1420 ARVO MX," made by Perkin Elmer Corp.).

### (Final concentrations of test substances)

Negative control: none (1 v/v% DMSO only)
Test group 1: cu538-1, 0.003 mM (in 1 v/v% DMSO)
Test group 2: cu538-2, 0.003 mM (in 1 v/v% DMSO)
Positive control: Latrunclin A (LatA), 0.0001 mM (in 1 v/v% DMSO)

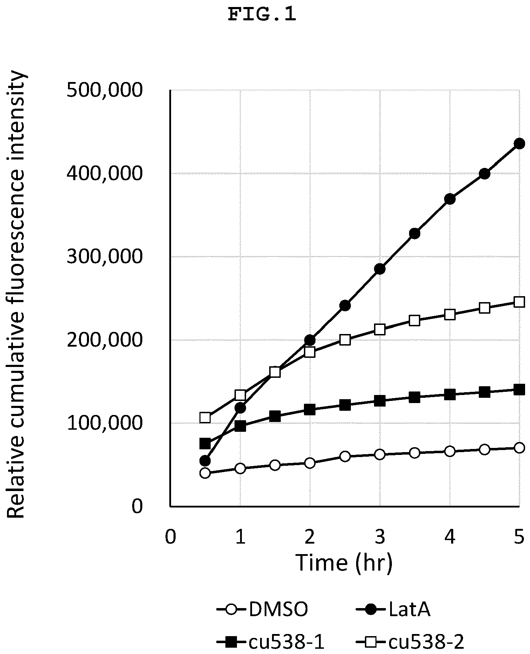

The results are shown in FIG. 1.

As indicated in FIG. 1, in the negative control, permeation of the FD4 into the receiver solution was restricted, the FD4 being the marker substance having a molecular weight of 4000. However, when Latrunclin A (LatA), which is an actin polymerization inhibitor, was added as a positive control, a continuous increase/accumulation in the amount of marker substance permeating the receiver solution was observed over the course of five hours after the start of testing. It is known that actin polymerization inhibitors irreversibly relax cell bonding structures.

By contrast, at a point in time two hours after the start of testing, adding "cu538-1" and "NY-9" had resulted in an increase by about a factor of four in the amount permeating the receiver solution with respect to the negative control, and adding "cu538-2" had resulted in an increase by about a factor of eight with respect to the negative control. However, in the period from two hours to five hours after the start of testing, substantially no increase/accumulation in the amount permeating the receiver solution was observed.

It is considered that, although adding "cu538-1", "cu538-2", or "NY-38" accelerated permeation of substances in the paracellular pathway, this effect was reversible; specifically, the normal state of tight junctions and other cell bonding structures recovered quickly, and no further increase/accumulation in the amount of the substances permeating the paracellular pathway was observed.

### <Test example 2>

A test to evaluate permeation by substances was conducted in the same manner as in test example 1, except that the test substances were as indicated below.

### (Final concentrations of test substances)

Negative control: none (1 v/v% DMSO only)
Test group 3: cu538-2, 0.003 mM (in 1 v/v% DMSO)
Test group 4: NY-38, 0.003 mM (in 1 v/v% DMSO)
Positive control: Latrunclin A (LatA) 0.0001 mM (in 1 v/v% DMSO)

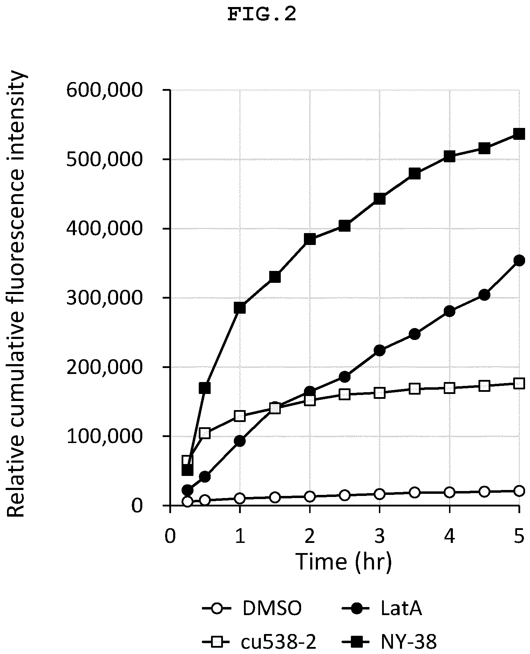

The results are shown in FIG. 2.

As indicated in FIG. 2, a cell-layer-permeation-promoting effect was found for "NY-38" as well. This effect was more remarkable than that for "cu538-2," for which the cell-layer-permeation-promoting effect was elucidated in test example 1.

### <Test example 3>

A test to evaluate permeation by substances was conducted in the same manner as in test example 1, except that the test substances were as indicated below.

### (Final concentrations of test substances)

Negative control: none (1 v/v% DMSO only)
Test group 5: cu538-2, 0.003 mM (in 1 v/v% DMSO)
Test group 6: cu592, 0.003 mM (in 1 v/v% DMSO)

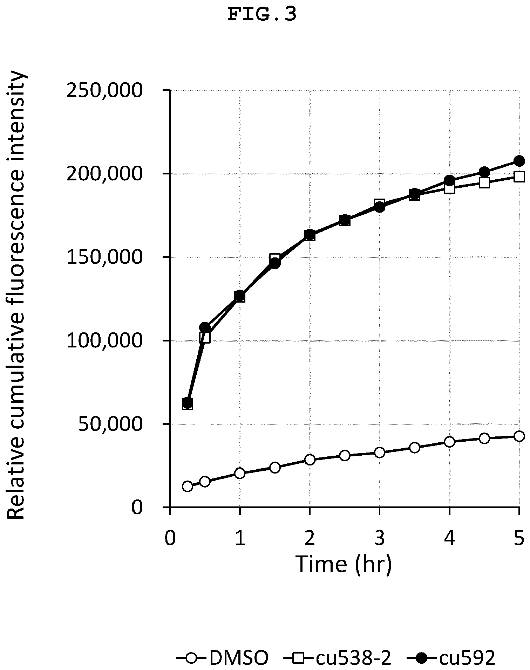

The results are shown in FIG. 3.

As indicated in FIG. 3, "cu592," which is an alanine scan form of the "cu538-2" for which the cell-layer-permeation-promoting effect was elucidated in test example 1, also was found to have an effect equivalent to that of the "cu538-2."

### <Test example 4>

A test to evaluate permeation by substances was conducted in the same manner as in test example 1, except that the test substances were as indicated below.

### (Final concentrations of test substances)

Negative control: none (1 v/v% DMSO only)
Test group 7: cu538-2, 0.003 mM (in 1 v/v% DMSO)
Test group 8: cu605, 0.003 mM (in 1 v/v% DMSO)
Test group 9: cu619, 0.003 mM (in 1 v/v% DMSO)

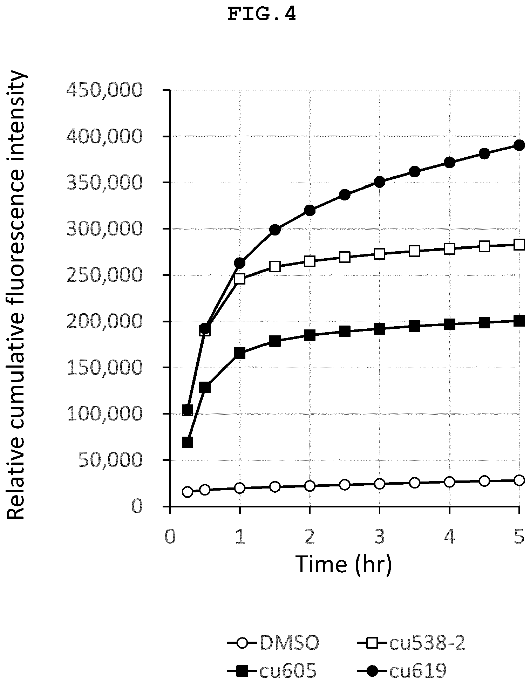

The results are shown in FIG. 4.

As indicated in FIG. 4, "cu605" and "cu619," which are alanine scan forms of the "cu538-2" for which the cell-layer-permeation-promoting effect was elucidated in test example 1, also were found to have an effect. The cell-layer-permeation-promoting effect of "cu619" in particular was more remarkable than that for "cu538-2."

## Claims

1. A cell layer permeation promoter comprising a compound represented by general formula (1). 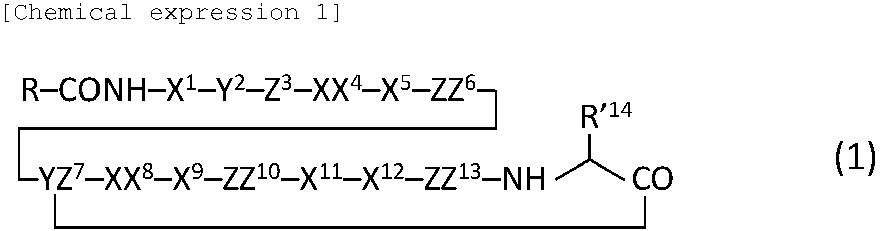
(In general formula (1): R is a C1-9 alkyl group optionally having a substituent;
X is leucine or a conservative substitute therefor, and is more preferably leucine, isoleucine, norleucine, tert-butyl alanine, tert-leucine, valine, cyclohexyl glycine or cyclohexyl alanine, or alanine;
XX is valine or a conservative substitute therefor, and is more preferably valine, isoleucine, norleucine, tert-butyl alanine, tert-leucine, leucine, cyclohexyl glycine or cyclohexyl alanine, or alanine;
Y is glutamic acid or a conservative substitute therefor, and is more preferably glutamic acid or aspartic acid, or alanine;
Z is glutamine or a conservative substitute therefor, and is more preferably glutamine or asparagine, or alanine;
ZZ is a discretionary α-amino acid, and is more preferably glutamine or alanine;
YZ is serine, threonine, homoserine, diaminopropanoic acid, or diaminobutyric acid; and
R' is sec-butyl or a conservative substitute therefor, and is more preferably sec-butyl, isopropyl, isopentyl, n-butyl, tert-butyl, cyclohexyl, or cyclohexyl methyl.)

2. The cell layer permeation promoter according to claim 1, wherein the compound represented by general formula (1) is a compound represented by general formula (2). 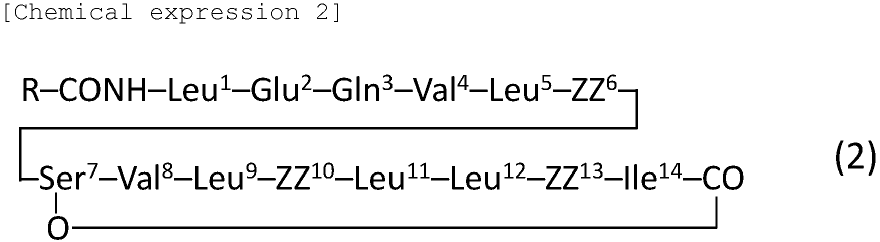
(In general formula (2): R is a C1-9 alkyl group optionally having a substituent; and
ZZ is a discretionary α-amino acid, and is more preferably glutamine or alanine.)

3. A composition for facilitating drug absorption that contains the cell layer permeation promoter according to claim 1 or 2, said composition being for facilitating absorption of a drug into a living body.

4. A pharmaceutical composition that contains the cell layer permeation promoter according to claim 1 or 2 and furthermore contains a drug to be absorbed into a living body.
